(19) Europäisches Patentamt
European Patent Office
Office européen des brevets

(11) EP 1 754 439 A1

(12) **EUROPEAN PATENT APPLICATION**

(43) Date of publication:
**21.02.2007 Bulletin 2007/08**

(51) Int Cl.:
***A61B 5/022*** *(2006.01)*

(21) Application number: **06016580.0**

(22) Date of filing: **08.08.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **15.08.2005 JP 2005235148**

(71) Applicant: **Omron Healthcare Co., Ltd.**
**Kyoto 615-0084 (JP)**

(72) Inventors:
- **Sawanoi, Yukiya**
  **Omron Healthcare Co., Ltd.**
  **Kyoto-shi**
  **Kyoto 615-0084 (JP)**
- **Kishimoto, Hiroshi**
  **Omron Healthcare Co., Ltd.**
  **Kyoto-shi**
  **Kyoto 615-0084 (JP)**
- **Tanaka, Takahide**
  **Omron Healthcare Co., Ltd.**
  **Kyoto-shi**
  **Kyoto 615-0084 (JP)**
- **Eda, Kenji**
  **Omron Healthcare Co., Ltd.**
  **Kyoto-shi**
  **Kyoto 615-0084 (JP)**
- **Miyata, Kiichiro**
  **Omron Healthcare Co., Ltd.**
  **Kyoto-shi**
  **Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(54) **Electronic blood pressure monitor, and blood pressure measurement data processing apparatus and method**

(57) In an electronic blood pressure monitor (1), a data storing portion (30) stores data of a blood pressure obtained by blood pressure measurement in a memory (12) in association with a condition at the time of the blood pressure measurement. An average value calculation portion (23) calculates average blood pressure data based on a blood pressure data group measured after getting up in the memory (12), and also calculates average blood pressure data based on a blood pressure data group measured before going to bed in the memory (12). A display control portion (25) displays a relation between the calculated average blood pressure data on a display portion (4) in a prescribed display manner.

FIG.1

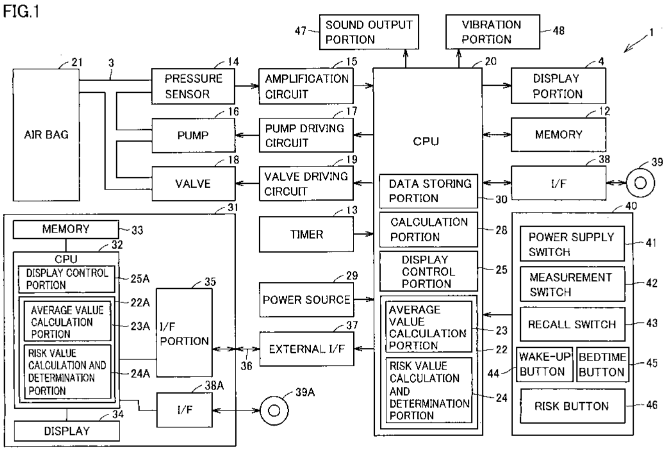


EP 1 754 439 A1

**Description**

BACKGROUND OF THE INVENTION

Field of the Invention

The present invention relates to an electronic blood pressure monitor and an apparatus and method for processing blood pressure measurement data, and particularly relates to an electronic blood pressure monitor and a blood pressure measurement data processing apparatus and method improved in display function of blood pressure measurement results.

Description of the Background Art

Currently, devices for measuring blood pressure at home are widely used. The blood pressure monitors for household use are provided with a function of notifying the user of the event that a one-time measurement value has exceeded a predetermined value, with a buzzer or a display, and a function of recording and displaying measurement values measured at different times.

The blood pressure is one of indices for analyzing the circulatory diseases. Performing risk analysis based on the blood pressure is effective for preventing cardiovascular diseases such as cerebral apoplexy, cardiac failure, and myocardial infarction. Especially the morning hypertension, an increased blood pressure in the early morning, has relation with cardiac diseases and strokes. Among the morning hypertension, the symptom that the blood pressure rapidly increases during the time after one to one and half hours from getting up, called "morning surge", has been found to have a cause-effect relationship with the cerebral apoplexy. Accordingly, it is useful to recognize the correlation between the time (living habit) and the change in blood pressure for risk analysis of the cardiovascular diseases.

Based on the foregoing, the blood pressure values measured are stored in a memory in association with measurement time information and/or measurement conditions. For example, a blood pressure monitor calculating an average value of blood pressure values measured during each of certain time zones, such as the morning time zone, the evening time zone and the like, and calculating and displaying a risk value based on the calculated results, is proposed (see Japanese Patent Laying-Open No. 2004-261452).

Further, the blood pressure monitor for household use may have a function of displaying a risk in the following manner. When it is determined that the blood pressure measurement value has exceeded a reference value, display of the numerical value by a segment LCD (Liquid Crystal Display) may be made to blink, or another indicator may be displayed (display using segment LCD, lighting or blinking of LED (Light Emitting Diode)) (see Japanese Patent Laying-Open Nos. 2000-175873 and 2004-121632).

With the invention disclosed in Japanese Patent Laying-Open No. 2004-261452, for example, a difference between an average value of the morning blood pressure values and an average value of the evening blood pressure values is displayed in two dimensions, and a determination value is provided for each of the average values and the difference value. It however fails to disclose a method for appropriately displaying the calculated risk.

With the inventions disclosed in Japanese Patent Laying-Open Nos. 2000-175873 and 2004-121632, although determination for a one-time measurement value as well as determination for an average value of measurements of several times are made, determination of the morning hypertension for preventing a risk of cardiovascular diseases is not made.

SUMMARY OF THE INVENTION

An object of the present invention is to provide an electronic blood pressure monitor and a blood pressure measurement data processing apparatus and method capable of intelligibly displaying the relation between the blood pressures measured in different measurement conditions or the relation of the blood pressure measured under a certain type of measurement condition with a reference blood pressure.

To achieve the above object, an electronic blood pressure monitor according to an aspect of the present invention stores data of a blood pressure obtained by blood pressure measurement in a memory unit or a memory in association with a type of a measurement condition at the time of the blood pressure measurement. It reads a blood pressure data group measured under the measurement condition of a first type and a blood pressure data group measured under the measurement condition of a second type different from the first type from the memory unit or the memory. It displays representative blood pressure data of one of the read blood pressure data groups on a display unit, and further displays a relation between the read blood pressure data groups on the display unit in a prescribed display manner. The relation includes a high/low relation between the blood pressures indicated by the representative blood pressure data of the respective blood pressure data groups.

Accordingly, the relation between the blood pressures measured under the different measurement conditions can be displayed. Further, for the blood pressures indicated by their representative blood pressure data, information as to whether one of them is higher or lower than the other of them can also be provided.

An electronic blood pressure monitor according to another aspect of the present invention stores data of a blood pressure obtained by blood pressure measurement in a memory unit or a memory in association with a type of a measurement condition at the time of the blood pressure measurement. It reads one of a blood pressure data group measured under the measurement condition of a first type and a blood pressure data group measured under the measurement condition of a second type different from the first type from the memory unit or the memory. It displays representative blood pressure data of the read blood pressure data group on a display unit, and further displays a relation between the read blood pressure data group and reference blood pressure data on the display unit in a prescribed display manner. The relation includes a high/low relation between a blood pressure indicated by the representative blood pressure data of the read blood pressure data group and a blood pressure indicated by the reference blood pressure data.

An electronic blood pressure monitor according to yet another aspect of the present invention stores data of a blood pressure obtained by blood pressure measurement in a memory unit or a memory in association with a type of a measurement condition at the time of the blood pressure measurement. It reads both of a blood pressure data group measured under the measurement condition of a first type and a blood pressure data group measured under the measurement condition of a second type different from the first type from the memory unit or the memory. It displays representative blood pressure data of one of the read blood pressure data groups on a display unit. It further displays a relation between the other of the read blood pressure data groups and reference blood pressure data on the display unit in a prescribed display manner. The relation includes a high/low relation between a blood pressure indicated by the representative blood pressure data of the other blood pressure data group and a blood pressure indicated by the reference blood pressure data.

An electronic blood pressure monitor according to yet another aspect of the present invention stores data of a blood pressure obtained by blood pressure measurement in a memory unit or a memory in association with a type of a measurement condition at the time of the blood pressure measurement. It reads both of a blood pressure data group measured under the measurement condition of a first type and a blood pressure data group measured under the measurement condition of a second type different from the first type from the memory unit or the memory. It displays representative blood pressure data of one of the read blood pressure data groups on a display unit. It further displays, for each of the read blood pressure data groups, a relation with reference blood pressure data, separately on the display unit in a prescribed display manner. The relation includes a high/low relation between a blood pressure indicated by the representative blood pressure data of each of the read blood pressure data groups and a blood pressure indicated by the reference blood pressure data.

Accordingly, the relation between the blood pressure data group measured under a certain type of measurement condition and reference blood pressure data can be displayed. Further, information as to whether the blood pressure of the representative blood pressure data of the blood pressure data group measured under a certain type of measurement condition is higher or lower than a reference blood pressure can also be provided.

In an electronic blood pressure monitor according to yet another aspect of the present invention, data of a blood pressure measured by a blood pressure measurement unit is stored in a memory unit in association with a type of a measurement condition at the time of blood pressure measurement. A blood pressure data group measured under the measurement condition of a first type and a blood pressure data group measured under the measurement condition of a second type different from the first type are read from the memory unit, and blood pressure data respectively representing the read blood pressure data groups are displayed on a display unit at the same time. Accordingly, it is possible to confirm the relation between the blood pressures measured under the different measurement conditions.

Preferably, suggestion of the high/low relation of the blood pressures is given by displaying a difference between the blood pressures using a bar-shaped indicator. Accordingly, the high/low relation of the blood pressures as well as the information of difference therebetween can be provided.

Preferably, it is controlled such that display color of blood pressure data is changed, the blood pressure data is displayed in blinking mode, a prescribed pattern or pictorial symbol (hereinafter, referred to as "icon") is displayed or not displayed (presence/absence of the icon), a backlight is lighted or not lighted, the color of emission of light is changed, or the backlight is made to blink or not to blink, in accordance with the high/low relation.

Accordingly, information as to whether one of the blood pressures of the blood pressure data having the high/low relation is higher or lower than the other can be provided in an intelligible manner with a simple display. As a result, only a simple function is required for the display unit, which can restrict the cost of the electronic blood pressure monitor.

Preferably, the control unit has a reference comparison unit that compares the blood pressure data representing the blood pressure data group measured under the measurement condition of a prescribed type with a reference blood pressure data for use in blood pressure evaluation. Accordingly, the comparison result by the reference comparison unit can be used to obtain the high/low relation.

Preferably, the high/low relation is notified using vibration or a sound. Accordingly, the high/low relation can be presented, not only by the visual information by means of display, but also using vibration or auditory information.

Preferably, the measurement conditions of the different types refer to measurement in the evening and measurement in the morning. Alternatively, they refer to measurement within a prescribed period after getting up and measurement within a prescribed period before going to bed.

Accordingly, it is possible to help the user to determine the morning hypertension by displaying the high/low relation between the blood pressure in the morning and the blood pressure in the evening, or the high/low relation between the blood pressure after getting up and the blood pressure before going to bed.

Preferably, the measurement condition of the second type refers to measurement conducted at least one day apart from the time indicated by the measurement condition of the first type.

According to the present invention, it is possible to display the relation between the blood pressures measured under the different measurement conditions.

The foregoing and other objects, features, aspects and advantages of the present invention will become more apparent from the following detailed description of the present invention when taken in conjunction with the accompanying drawings.

## BRIEF DESCRIPTION OF THE DRAWINGS

Fig. 1 is a functional configuration diagram of an electronic blood pressure monitor according to an embodiment.

Fig. 2 is an overall view of the electronic blood pressure monitor according to the embodiment.

Figs. 3A and 3B show storage examples of measurement data in the electronic blood pressure monitor according to the embodiment.

Figs. 4-6 are flowcharts illustrating examples of measurement procedure of the electronic blood pressure monitor according to the embodiment.

Figs. 7-10, 11A-11D, 12, 13A-13D, 14-23, 24A-24E, 25-29, 30A-30E, 31-34, 35A, 35B and 36A-36C show display examples of measurement results according to the embodiment.

## DESCRIPTION OF THE PREFERRED EMBODIMENTS

Hereinafter, an embodiment of the present invention will be described in detail with reference to the drawings. In the following, the same portions and components have the same reference characters allotted, and their designation and function are identical. Therefore, detailed description thereof will not be repeated.

### (Configuration)

A functional configuration of an electronic blood pressure monitor according to an embodiment is shown in Fig. 1, and its overview is shown in Fig. 2.

Referring to Fig. 2, an electronic blood pressure monitor 1 according to the present embodiment includes a cuff 5 fitted to a blood pressure measurement site of a subject and pressurized by an air pressure, and an air tube 3 connecting a blood pressure monitor main body 2 with cuff 5.

Blood pressure monitor main body 2 includes a display portion 4 provided for the subject to confirm a displayed content, a power supply switch 41, a measurement switch 42, a recall switch 43, a wake-up button 44, a bedtime button 45, and a risk button 46, provided for the subject to be manipulable from the outside.

Power supply switch 41 is manipulated for turning ON/OFF the power source of blood pressure monitor main body 2. Measurement switch 42 is manipulated for designating start of blood pressure measurement. Recall switch 43 is manipulated for reading a temporarily stored measurement result and displaying the same on a display portion 4. Wake-up button 44 is manipulated in the case where blood pressure measurement is conducted after getting up, for example within two hours after getting up. Bedtime button 45 is manipulated in the case where blood pressure measurement is conducted before going to bed, e.g., within two hours before going to bed. Letters of "after getting up" and "before going to bed" are printed on wake-up button 44 and bedtime button 45, respectively. Risk button 46 is manipulated for designating calculation of a risk value based on the stored blood pressure measurement results.

Referring to Fig. 1, electronic blood pressure monitor 1 includes an air bag 21 contained in cuff 5, a pressure sensor 14 that outputs a change in pulse pressure at the measurement site detected via air bag 21 as a pulse wave signal, an amplification circuit 15 that amplifies a voltage signal indicating the pressure output from pressure sensor 14, a pump 16 and a valve 18 for adjusting a pressurizing (air pressure) level of air bag 21, a pump driving circuit 17 driving pump 16, a valve driving circuit 19 for adjusting opening/closing of valve 18, a display portion 4, a memory 12, a manipulation portion 40, a timer 13 that performs time-counting operation and outputs the time data, a power source 29, a sound output portion 47, a vibration portion 48, and a CPU (Central Processing Unit) 20 that controls the respective portions.

CPU 20 has, therein, a memory (not shown) for temporarily storing data, a risk processing portion 22, a display control portion 25, a calculation portion 28 calculating blood pressure and pulse rate, and a data storing portion 30 having a function of storing blood pressure measurement data in memory 12. CPU 20 has a function of processing the blood pressure measurement data. Display control portion 25 controls display on display portion 4. In the present embodiment, the functions of risk processing portion 22, display control portion 25, calculation portion 28, and data storing portion 30 are realized as CPU 20 reads and executes prescribed programs from memory 12. It is noted that a part or all of the functions of the programs may be configured with hardware such as a circuit.

Air bag 21 is connected to pressure sensor 14, pump 16 and valve 18 via air tube 3. Herein, power source 29, supplying power for driving the respective portions, is formed of a battery or a commercial power source. Calculation portion 28 calculates a blood pressure value, a pulse rate and the like based on the pulse signal input from amplification circuit 15.

Manipulation portion 40 has the switches shown in Fig. 2, including power supply switch 41, measurement switch 42, recall switch 43, wake-up button 44, bedtime button 45, and risk button 46.

Memory 12 stores various data including the measurement result data, and various programs.

In the above-described configuration, at the time of blood pressure measurement, calculation portion 28 converts the pulse signal (pressure signal) output from amplification circuit 15 to digital data, and then applies a prescribed algorithm to the data to calculate a systolic blood pressure (maximum blood pressure), a diastolic blood pressure (minimum blood pressure), and a pulse rate. Conventionally known procedures can be used for such calculation, and thus, detailed description thereof is not provided here.

Electronic blood pressure monitor 1 has an external I/F (Interface) 37 for connecting an external apparatus via communication. External I/F 37 connects an external computer 31 via a communication line 36. Computer 31 may be a computer of a subject, or a computer at a doctor's office. Communication line 36 may be wired or wireless. CPU 20 transmits measurement data stored in memory 12 via external I/F 37 to computer 31. Computer 31 has a CPU 32, a memory 33, a display 34, and an I/F portion 35 for connecting a communication line. CPU 32 stores the blood pressure measurement data received from electronic blood pressure monitor 1 (i. e., contents of memory 12) in memory 33, in a similar manner as in memory 12. CPU 32 processes the blood pressure measurement data stored in memory 33 in a manner similar to that of CPU 20.

As shown in Figs. 1 and 2, in electronic blood pressure monitor 1 of the present embodiment, measurement conditions being blood pressure measurement time zones according to the daily rhythm (after getting up, before going to bed) are allocated in advance to wake-up button 44 and bedtime button 45, respectively. The blood pressure measurement result is stored in a prescribed area in memory 12, in association with the measurement condition allocated to the button (either wake-up button 44 or bedtime button 45) manipulated at the time of the blood pressure measurement. Storage examples of the blood pressure measurement results in memory 12 in this case are shown in Figs. 3 A and 3B.

In Fig. 3A, memory 12 has areas 26 and 27 provided in advance corresponding to the respective measurement conditions. The blood pressure measurement result is stored in the area corresponding to the measurement condition of the manipulated button, i.e., in the area (either area 26 or area 27) corresponding to the measurement time zone (before going to bed or after getting up), in units of records R. Record R includes data T indicating a measurement time input from timer 13, systolic blood pressure data SBP indicating a systolic blood pressure, diastolic blood pressure data DBP indicating a diastolic blood pressure, and pulse rate data PLS indicating a pulse rate. The storage manner of these four data items is not restricted to the one using records R. All that is needed is that every time measurement is conducted, the data are stored in a corresponding area in an associated manner.

For measurement time data T, CPU 20 inputs data of the measurement time (time at start or end of measurement) counted by timer 13, and converts it into measurement time data T (year, month, day, hour, minute, day of the week), which is stored in record R. As such, it may be possible to determine which condition of before going to bed (evening) or after getting up (morning) is to be associated with measurement result data based on measurement time data T, and, based on the determined result, store the relevant measurement result data in an area in memory 12 corresponding to the determined condition. Such determination based on measurement time data T may be used in place of the manipulation of wake-up button 44 and bedtime button 45. Alternatively, it may be used in the case where wake-up button 44 and bedtime button 45 are not provided. Herein, for simplification of explanation, it is assumed that measurement data are stored in areas in memory 12 corresponding to the measurement conditions in accordance with manipulation of wake-up button 44 and bedtime button 45, as shown in Fig. 3A.

The method of associating the measurement results with the measurement conditions in memory 12 is not limited to the one shown in Fig. 3A. For example, it may be as shown in Fig. 3B. In Fig. 3B, a record Ri (i = 1, 2, 3, ..., m) storing a measurement result such as a blood pressure value and information of measurement condition as a pair is generated every time blood pressure measurement is conducted, and the generated record Ri is stored in memory 12. Record Ri stores measurement time data Ti, systolic blood pressure data SBPi, diastolic blood pressure data DBPi, pulse rate data PLSi, and measurement condition data C1 or C2. Measurement condition data C1 and C2 each indicate measurement condition information shown by wake-up button 44 or bedtime button 45 manipulated at the time of blood pressure measurement, i.e., the measurement time zone (before going to bed or after getting up).

(Calculation of Risk Value)

In the present embodiment, a risk of cardiovascular system is calculated based on the measured blood pressure values stored in memory 12. More specifically, CPU 20 calculates the risk value based on a program for calculating a cardiovascular risk value pre-stored in memory 12. The processing according to this program corresponds to the function of risk processing portion 22 of CPU 20. Risk processing portion 22 has an average value calculation portion 23 and a risk value calculation and determination portion 24.

For calculation of a risk value, firstly, average value calculation portion 23 reads blood pressure values stored in memory 12, and calculates an average of the blood pressure values for each of areas 26 and 27 in Fig. 3A. That is, for each blood pressure data group consisting of a plurality of blood pressure data items measured under the same measurement condition, it calculates an average value, for example, as a representative value of the group. Herein, it is assumed that m records R are stored in each of areas 26 and 27. Average blood pressure values are calculated using the following expressions. It is noted that in the case of m = 1, the representative value of the blood pressure data group corresponds to the measurement data indicated by the one (m = 1) record R included in the group.

Average of SBP measured after getting up = (systolic blood pressure data SBP1 measured after getting up + systolic blood pressure data SBP2 measured after getting up + ... + systolic blood pressure data SBPm measured after getting up) / m (where m = 1, 2, 3, ...).

Average of DBP measured after getting up = (diastolic blood pressure data DBP1 measured after getting up + diastolic blood pressure data DBP2 measured after getting up + ... + diastolic blood pressure data DBPm measured after getting up) / m (where m = 1, 2, 3, ...).

Average of SBP measured before going to bed = (systolic blood pressure data SBP 1 measured before going to bed + systolic blood pressure data SBP2 measured before going to bed + ... + systolic blood pressure data SBPm measured before going to bed) / m (where m = 1, 2, 3, ...).

Average of DBP measured before going to bed = (diastolic blood pressure data DBP1 measured before going to bed + diastolic blood pressure data DBP2 measured before going to bed + ... + diastolic blood pressure data DBPm measured before going to bed) / m (where m = 1, 2, 3, ...).

Average of PLS measured after getting up = (pulse rate data PLS 1 measured after getting up + pulse rate data PLS2 measured after getting up + ... + pulse rate data PLSm measured after getting up) / m (where m = 1, 2, 3, ...).

Average of PLS measured before going to bed = (pulse rate data PLS 1 measured before going to bed + pulse rate data PLS2 measured before going to bed + ... + pulse rate data PLSm measured before going to bed) / m (where m = 1, 2, 3, ...).

Risk value calculation and determination portion 24 determines the risk value based on the calculated results according to the above expressions. Specifically, if it is determined that average of SBP measured after getting up > 135 mmHg, or average of DBP measured after getting up > 85 mmHg, it is determined to be "morning hypertension". It is noted that the reference values for risk determination are defined, e.g., by Joint National Committee on Prevention, Detection, Evaluation, and Treatment of High blood Pressure in the U.S.A., or by Japanese Society of Hypertension as the reference values of home blood pressure for determination of high blood pressure. The reference value of systolic blood pressure SBP is 135 mmHg, and the reference value of diastolic blood pressure DBP is 85 mmHg. These reference values are pre-stored in an internal memory (not shown) of CPU 20.

Further, for calculation of the risk value, risk value calculation and determination portion 24 calculates an average value of blood pressure values measured in the time zone before going to bed and in the time zone immediately after getting up (hereinafter, referred to as "ME average") and a difference therebetween (hereinafter, referred to as "ME difference") according to the following expressions.

ME difference = average of SBP measured after getting up − average of SBP measured before going to bed

ME average = (average of SBP measured after getting up + average of SBP measured before going to bed) / 2

Risk value calculation and determination portion 24 uses these expressions to determine the risk value. For example, if it is determined that ME difference > 20 mmHg and ME average > 135 mmHg, it is determined to be "morning hypertension". Further, it is determined to be "morning hypertension" when ME difference > 20 mmHg, as shown in Japanese Patent Laying-Open No. 2004-261452. The reference values for such determination are pre-stored in an internal memory (not shown) of CPU 20.

Blood pressure values would vary affected by various factors. Thus, it is preferable to repeat blood pressure measurement in these time zones over a plurality of days and obtain average values for the respective time zones, which are then used for obtaining the ME average and ME difference for use in the risk determination. This improves precision in risk estimation.

(Measurement Procedure)

An operation of blood pressure measurement using electronic blood pressure monitor 1 of the present embodiment will be described hereinafter. The flowcharts in Figs. 4-6 are pre-stored in memory 12 as programs, which are read and executed by CPU 20. The blood pressure measurement operation according to the flowchart of Fig. 4 will now be described.

Firstly, when a subject winds cuff 5 around the measurement site and manipulates power supply switch 41 of electronic blood pressure monitor 1, a manipulation signal is applied to CPU 20. CPU 20, in response to the applied manipulation signal, controls power source 29 to start power supply to the respective portions (step ST (hereinafter, simply abbreviated as "ST") 1). Next, as the initialization processing of electronic blood pressure monitor 1, CPU 20 controls certain portions to evacuate the air within air bag 21 such that the output level of pressure sensor 14 is 0 mmHg (ST2).

Next, the subject manipulates a button corresponding to the measurement condition, i.e., either wake-up button 44 or bedtime button 45, to input the measurement condition (ST3). A different manipulation signal for each of the manipulated button is applied from manipulation portion 40 to CPU 20. CPU 20 determines which button was manipulated based on the applied manipulation signal, and temporarily stores in an internal memory (not shown) data of the measurement condition corresponding to the button indicated by the determined result.

Subsequently, the subject manipulates measurement switch 42, and a signal indicating the manipulation is applied to CPU 20. CPU 20 detects that initiation of blood pressure measurement has been instructed, based on the applied manipulation signal (ST4). The subsequent blood pressure measurement is thus started. When measurement switch 42 is manipulated, CPU 20 inputs a current time from timer 13, and temporarily stores the input time as measurement time data T in the internal memory (not shown).

Determination of the measurement condition based on the manipulation of wake-up button 44 or bedtime button 45 is carried out for example in the following manner. Memory 12 pre-stores, for each switch provided at manipulation portion 40, data of a manipulation signal indicating that the corresponding switch was manipulated and data of content of designation indicated by the relevant manipulation signal in association with each other. Thus, CPU 20 can retrieve the associated data within memory 12 based on the manipulation signal applied from manipulation portion 40, and read the data of the designation content corresponding to the manipulation signal. By interpreting the read data of designation content, CPU 20 can determine the event that the measurement condition was designated and also determine the designated measurement condition (whether measurement before going to bed or measurement after getting up).

In response to the manipulation of measurement switch 42 above, CPU 20 controls certain portions to increase the pressure within air bag 21 to about the systolic blood pressure of the subject + 40 mmHg (ST5), and then gradually decreases the pressure within air bag 21 (ST6). It is assumed that the systolic blood pressure of the subject is input by the subject in advance. During this pressure-decreasing process, the pressure within air bag 21 is detected by pressure sensor 14. Calculation portion 28 of CPU 20 calculates the (systolic and diastolic) blood pressure values and the pulse rate based on the detected pressure, and temporarily stores them in an internal memory of CPU 20 (ST7). At this time, display control portion 25 displays the calculated blood pressure values and pulse rate on display portion 4 (ST8). The pressure increasing and decreasing processes for the blood pressure measurement are similar to those of a conventional electronic blood pressure monitor. Although it is herein configured to measure the blood pressure during the pressure-decreasing process, it may be measured during the pressure-increasing process.

When the calculation and display of the blood pressure and pulse rate are finished, data storing portion 30 of CPU 20 searches memory 12 based on the data (stored in the internal memory) of measurement condition designated by the button manipulated in ST3, and specifies an area corresponding to the measurement condition. It then generates a new record R storing the measurement results (blood pressure value data SBP and DBP as well as pulse rate data PLS) temporarily stored in the internal memory of CPU 20 and measurement time data T, and registers (stores) the generated record R in the specified area in memory 12 (ST9). The blood pressure measurement data is thus stored in memory 12 in association with the measurement condition. A series of blood pressure measurement operations are thus completed.

In the procedure of Fig. 4, the processing of inputting the measurement condition (ST3) is carried out at the time of start of blood pressure measurement. Alternatively, the input of the measurement condition (ST8a) may be carried out when storing the measurement result data in memory 12 (ST9), as shown in Fig. 5. Otherwise, the processing in Fig. 5 is identical to that of Fig. 4.

Further, at the end of blood pressure measurement, the measurement result is temporarily stored in the internal memory of CPU 20. Thus, the subject can manipulate manipulation portion 40 after completion of measurement to read the measurement result data from the internal memory to display the read data on display portion 4. The subject may manipulate wake-up button 44 or bedtime button 45 after confirming the measurement result thus displayed, to input (designate) the measurement condition.

In the processing in Figs. 4 and 5, all the measurement result data are stored in memory 12. Alternatively, the measurement result data may be selectively stored in memory 12 by inputting the measurement condition.

In the case where calculation and display of a risk value is to be carried out following the blood pressure measurement processing in Fig. 5, the procedure shown in Fig. 6 is carried out. In the procedure of Fig. 6, after the processing of ST1-ST9 in Fig. 5 are carried out in a similar manner, risk processing portion 22 of CPU 20 determines whether a prescribed number of data items required for calculation of risk value, e.g., at least one data item, have been recorded in each area in memory 12 (ST10). If not (NO in ST10), a series of processing are terminated. If it is determined that the prescribed number of data items required for calculation of risk value are recorded in each area (YES in ST10), the measurement result data are read from memory 12, and based on the read data, an average value is calculated by average value calculation portion 23 according to the above-described expression, or calculation and determination of the risk value is carried out by risk value calculation and determination portion 24 (ST13). The result of calculation, or the result of determination, is displayed on display portion 4 by display control portion 25 (ST15). Thereafter, a series of processing are completed.

Although processing of risk value calculation/display is carried out following the blood pressure measurement in Fig. 6, it is not limited to such continuous processing. Calculation and display of the risk value may be started in response to manipulation of risk button 46 in manipulation portion 40 by the subject.

The processing in ST13 and ST15 in Fig. 6 may be configured to be carried out at computer 31. That is, the measurement data stored in memory 12 of electronic blood pressure monitor 1 as shown in Fig. 3A or 3B may be read by CPU 20, and transmitted to computer 31 via communication line 36. CPU 32 of computer 31 may receive and store the measurement data in a memory 33, and carry out the processing shown in ST13 and ST15 based on the stored measurement data. It may then display the result on a display 34 having the function equivalent to that of display portion 4.

In this case, it is assumed that CPU 32 has a risk processing portion 22A including an average value calculation portion 23A and a risk value calculation and determination portion 24A as well as a display control portion 25A, having the functions equivalent to those of risk processing portion 22 including average value calculation portion 23 and risk value calculation and determination portion 24 as well as display control portion 25. Specifically, it is assumed that it executes programs equivalent to risk processing portion 22A including average value calculation portion 23A and risk value calculation and determination portion 24A as well as display control portion 25A. In such a case, computer 31 is in charge of risk calculation/determination and display, while electronic blood pressure monitor 1 is in charge of blood pressure measurement, so that the load can be distributed therebetween.

Further, communicating with computer 31 at a doctor's home allows the doctor to receive the blood pressure data of the subject living at a long distance, and advantageously use the data for diagnosis and treatment.

It is noted that the measurement data may be supplied to computer 31, not only via communication line 36, but also by using various recording media such as magnetic and optical disks.

The above-described processing functions are realized by programs. Such programs are stored in computer readable recording media. In the present embodiment, the memories originally required for performing processing in electronic blood pressure monitor 1 or computer 31 shown in Fig. 1, e.g., memory 12 and memory 33 themselves, may be the program recording media. Alternatively, they may be CD-ROM (Compact Disk-Read Only Memory) 39 and 39A mounted to the apparatuses in a detachable manner. In this case, electronic blood pressure monitor 1 and computer 31 are provided with I/F 38 and 38A serving as program reading devices, such as CD-ROM devices as external memory devices, and the recording media, CD-ROM 39 and 39A, are inserted therein. In any case, the program stored in the recording medium is accessed and executed by CPU 20 or CPU 32.

Herein, the above-described program medium is a recording medium configured to be separable from electronic blood pressure monitor 1 or computer 31, which may be a disk-type recording medium, such as a magnetic disk like a flexible disk, and an optical disk like a CD-ROM / MO (Magnetic Optical Disk) / MD (Mini Disk) / DVD (Digital Versatile Disk), or may be a card-type recording medium, such as an IC card (including memory card) / optical card.

Further, in the present embodiment, communication line 36 may be one of communication networks including the Internet. In such a case, programs may be downloaded to memories 12 and 33 of electronic blood pressure monitor 1 and computer 31 via communication line 36.

(Display Examples of Risk Values)

Hereinafter, various display examples of risk values on display portion 4 by display control portion 25, or various display examples of risk values on display 34 by display control portion 25A, in ST15 will be shown. In the display examples in each figure, "SBP" represents a systolic blood pressure, "DBP" represents a diastolic blood pressure, and "PLS" represents a pulse rate.

(Display Example 1)

In Fig. 7, display portion 4 includes display areas 51 and 52 for displaying measurement data after getting up and measurement data before going to bed, side by side simultaneously. In display area 51, average data of SBP measured after getting up 61, average data of DBP measured after getting up 62, and average data of PLS measured after getting up 63 obtained according to the above expressions by average value calculation portion 23 are displayed, and side by side therewith, in display area 52, average data of SBP measured before going to bed 71, average data of DBP measured before going to bed 72, and average data of PLS measured before going to bed 73 obtained according to the above expressions by average value calculation portion 23 are displayed. As such, the measurement data in display areas 51 and 52 are displayed in a manner associated with each other.

Risk value calculation and determination portion 24 compares the data displayed on display areas 51 and 52. Based on the comparison result, display control portion 25 may display the high/low relation therebetween in a prescribed manner, for example by changing the display color of the data of higher value to a prescribed color different from that of the data of lower value. For example, the value of average data of SBP measured after getting up 61 and the value of average data of SBP measured before going to bed 71 may be compared with each other, and all the data in the display area including the higher blood pressure may be displayed in a prescribed color. Fig. 8 is a display example involving color change when average data of SBP measured after getting up 61 is higher. The display manner is not limited to such display involving color change. It may be changed to display in blinking mode.

Further, after comparison between average data of SBP measured after getting up 61 and average data of SBP measured before going to bed 71, only the one indicating the higher blood pressure may be displayed in blinking mode. Alternatively, after comparing average data of DBP measured after getting up 62 with average data of DBP measured before going to bed 72, only the data indicating the higher blood pressure may be displayed in different color. Still alternatively, the data indicating higher blood pressure as a result of comparison between average data of SBP measured after getting up 61 and average data of SBP measured before going to bed 71, and the data indicating higher blood pressure as a result of comparison between average data of DBP measured after getting up 62 and average data of DBP measured before going to bed 72, may both be displayed in different color.

Risk value calculation and determination portion 24 further compares the values of blood pressure data in display areas 51 and 52 with reference values for use in risk determination (135 mmHg, 85 mmHg). Based on the comparison result, display control portion 25 may display whether the values are higher or lower than the reference values. For example, of display areas 51 and 52, all the data in the area including the blood pressure value higher than the reference value may be displayed in blinking mode. Alternatively, the blood pressure data (only the systolic blood pressure, only the diastolic blood pressure, or both) higher than the reference value(s) may be displayed in different color.

Further, the display manner may also be as follows. Display control portion 25 may control such that a backlight provided in advance at the background of data of blood pressure (systolic blood pressure, diastolic blood pressure, or both) determined to be high based on the above comparison result is changed to be different from the backlight of the blood pressure data determined to be low. For example, it may cause only the backlight of the higher blood pressure data to light or blink. Alternatively, it may cause the backlight of the blood pressure data determined to be high and the backlight of the blood pressure data determined to be low, to emit lights of different colors. It may also differentiate the backlights from each other by using different emission colors and blinking, or by changing the intervals of blinking.

In Fig. 9, only the representative values (data measured after getting up, consisting of average data of SBP measured after getting up 61, average data of DBP measured after getting up 62 and average data of PLS measured after getting up 63) calculated for the blood pressure data group read from one of areas 26 and 27, i.e., area 27 in this case, are displayed on display portion 4.

Risk value calculation and determination portion 24 compares the values indicated by the representative blood pressure data displayed on display portion 4 as in Fig. 9 with the reference values for use in risk determination (135 mmHg, 85 mmHg). Based on the comparison result, display control portion 25 displays on display portion 4 whether they are higher or lower than the reference values. For example, when the blood pressure data higher than the reference values are being displayed, the backlight of display portion 4 may be changed to notify that the blood pressure is higher than the reference value. Alternatively, only the backlight(s) of blood pressure data higher than the reference value(s) (only systolic blood pressure, only diastolic blood pressure, or both) may be changed to differ from the other backlight (s). The manner of change of the backlight may include turning off, turning on, or blinking. The color of emission of light may be changed, or the color of emission of light and blinking may both be utilized. The intervals of blinking may also be changed. It is noted that the same applies to the case where average value calculation portion 23 reads the data from area 26 out of areas 26 and 27.

In the display manner in Fig. 9, the type of measurement condition for which data are to be displayed may be designated by the user via manipulation portion 40, or it may be predetermined.

(Display Example 2)

In Fig. 10, display portion 4 includes display areas 51 and 53. In display area 51, for example, average data of SBP measured after getting up 61, average data of DBP measured after getting up 62 and average data of PLS measured after getting up 63 are displayed. In display area 53, the result of comparison of the average data being displayed on display area 51 with the reference values for risk determination (135 mmHg, 85 mmHg), conducted by risk value calculation and determination portion 24, may be displayed, or the result of comparison with the average values of the data measured before going to bed may be displayed. In display area 53, blocks 81-84 for showing the comparison results are displayed. The display examples of blocks 81-84 in display area 53 are shown in Figs. 11A-11D.

In the case of the display in Fig. 10, average value calculation portion 23 reads the measurement data from both areas 26 and 27 provided for different measurement conditions, and calculates the respective average values according to the above-described expressions. Display control portion 25 selects and displays the average data of either one measurement condition on display area 51. Although the average data for area 27 corresponding to measurement after getting up are displayed here, the average data of area 26 corresponding to measurement before going to bed may be selected. The type of measurement condition for which data are to be displayed on display area 51 of Fig. 10 may be designated by the user via manipulation portion 40, or may be predetermined.

In Fig. 11A, blocks 81-84 are displayed in reverse (or non-reverse) mode to show the comparison results between the risk determination reference values (135 mmHg, 85 mmHg) 80 and the blood pressure measurement values. Specifically, if it is determined as a result of comparison that average data of SBP measured after getting up 61 and average data of DBP measured after getting up 62 are higher than reference values 80 and average data of SBP measured before going to bed 71 and average data of DBP measured before going to bed 72 are lower than reference values 80, then the upper and lower blocks 81 and 82 corresponding to the morning (after getting up), sandwiching the reference values 80 therebetween, are both displayed in reverse mode (filled in). On the other hand, of the upper and lower blocks 83 and 84 corresponding to the evening (before going to bed), sandwiching the reference values 80 therebetween, block 84 lower than reference values 80 is displayed in reverse mode, while upper block 83 is displayed in non-reverse mode (in blank).

Whether the value is higher or lower than reference value 80 is determined by risk value calculation and determination portion 24. Specifically, if it is determined that a prescribed relation "[systolic blood pressure > 135 mmHg or diastolic blood pressure > 85 mmHg] or [systolic blood pressure > 135 mmHg and diastolic blood pressure > 85 mmHg]" is satisfied, it is evaluated that the blood pressure value is higher than a prescribed reference value 80. If the prescribed relation is not satisfied, it is evaluated that the blood pressure value is lower than the prescribed reference value 80. For the morning (after getting up), one or both of average data of SBP measured after getting up 61 and average data of DBP measured after getting up 62 are adapted for this prescribed relation. For the evening (before going to bed), one or both of average data of SBP measured before going to bed 71 and average data of DBP measured before going to bed 72 are adapted for the prescribed relation.

In Fig. 11B, risk value calculation and determination portion 24 compares the blood pressures measured after getting up and measured before going to bed, and based on the comparison result, display control portion 25 displays whether they are higher or lower using blocks 81-84. In Fig. 11B, since the value of average data of SBP measured after getting up 61 is higher than the value of average data of SBP measured before going to bed 71, upper and lower blocks 81 and 82 corresponding to the morning (after getting up) are displayed in reverse mode, and of the upper and lower blocks corresponding to the evening (before going to bed), lower block 84 is displayed in reverse mode to show the comparison result.

Further, in Figs. 11C and 11D, the average values of blood pressure measurement data after getting up and before going to bed are compared, and the high/low relation therebetween is displayed based on the comparison result, and in addition, the high/low relation of the average values with the prescribed reference values is displayed. In Fig. 11C, risk value calculation and determination portion 24 determines that average data of SBP measured after getting up 61 is higher than average data of SBP measured before going to bed 71 and that the average data measured after getting up is higher than reference value 80 based on the above-described prescribed relation. Based on the determined result, display control portion 25 displays upper and lower blocks 81 and 82 corresponding to the morning (after getting up) in reverse mode, and displays blocks 83 and 84 in blank. Similarly, in Fig. 11D, it is determined that average data of SBP measured after getting up 61 is higher than average data of SBP measured before going to bed 71 and that the average data measured after getting up is lower than reference value 80 based on the above-described prescribed relation. Thus, of upper and lower blocks 81 and 82 corresponding to the morning (after getting up), lower block 82 alone is displayed in reverse mode, and the remaining blocks are displayed in blank.

(Display Example 3)

Fig. 12 shows another display manner of blocks 81-84 in display area 53 of Fig. 10. The display manners of blocks 81-84 in display area 53 are shown in Figs. 13A-13D. The operation of average value calculation portion 23 is identical to that in the case of Fig. 10.

In Fig. 13A, a comparison result between the blood pressure measurement values and reference values 80 is shown. For example, assume the case where it is determined that the above-described prescribed relation is satisfied for one or both of average data of SBP measured after getting up 61 and average data of DBP measured after getting up 62 with respect to reference values 80 (i.e., the blood pressure after getting up is higher than reference value 80), and that the above-described prescribed relation is not satisfied for one or both of average data of SBP measured before going to bed 71 and average data of DBP measured before going to bed 72 with respect to reference values 80 (i.e., the blood pressure before going to bed is lower than reference value 80). In this case, of upper and lower blocks 81 and 82 corresponding to the morning (after getting up), sandwiching reference values 80 therebetween, upper block 81 is displayed in reverse mode (filled in), and of upper and lower blocks 83 and 84 corresponding to the evening (before going to bed), sandwiching reference values 80 therebetween, lower block 84 is displayed in reverse mode (filled in), and the remaining blocks are displayed in non-reverse mode (in blank) to show the determined (comparison) results.

Further, in Fig. 13B, the average values of blood pressure measurement data after getting up and before going to bed are compared, and their relation based on the comparison result is displayed using blocks 81-84. In the display example in Fig. 13B, for example, it is determined by comparison that the value of average data of SBP measured after getting up 61 is higher than the value of average data of SBP measured before going to bed 71. To display the determined result, upper block 82 out of blocks 81 and 82 corresponding to the morning (after getting up), and lower block 84 out of blocks 83 and 84 corresponding to the evening (before going to bed) are displayed in reverse mode (filled in), and the remaining blocks are displayed in non-reverse mode (in blank).

Figs. 13C and 13D show the manners of displaying both the comparison result of blood pressures after getting up and before going to bed, and the comparison result with respect to reference values 80. Fig. 13C shows the display example in the case where it is determined that average data of SBP measured after getting up 61 is higher than average data of SBP measured before going to bed 71 and that the above-described prescribed relation is satisfied for average data of SBP measured after getting up 61 and average data of DBP measured after getting up 62 with respect to reference values 80 (i.e., they are higher than reference values 80). Specifically, of upper and lower blocks 81 and 82 corresponding to the morning (after getting up), sandwiching reference values 80 therebetween, block 81 upper than reference values 80 is displayed in reverse mode (filled in), and the remaining blocks are displayed in non-reverse mode (in blank).

Further, Fig. 13D shows the display example in the case where it is determined that average data of SBP measured after getting up 61 is higher than average data of SBP measured before going to bed 71, that the average value of the blood pressure measurement data after getting up is higher than that before going to bed, and that the above-described prescribed relation is not satisfied for average data of SBP measured after getting up 61 and average data of DBP measured after getting up 62 with respect to reference values 80 (i.e., they are lower than reference values 80). Specifically, of upper and lower blocks 81 and 82 corresponding to the morning (after getting up), sandwiching reference values 80 therebetween, block 82 lower than reference values 80 is displayed in reverse mode (filled in), and the remaining blocks are displayed in non-reverse mode (in blank).

In Figs. 13B-13D, the blood pressure values after getting up and before going to bed are compared. The target of comparison is not restricted to the pair of average data of SBP measured after getting up 61 and average data of SBP measured before going to bed 71, but may be a pair of average data of DBP measured after getting up 62 and average data of DBP measured before going to bed 72.

In Display Examples 1-3 described above, the data of blood pressures measured under different measurement conditions are displayed along with their high/low relation. The measurement conditions of the data to be displayed are not limited to the averages of data measured after getting up and the averages of data measured before going to bed. For example, they may be data of current measurement and data of previous measurement, or may be an average value of data measured today and an average value of data measured yesterday. Further, they may be an average value of data measured on Monday and an average value of data measured on the other days of the week. Still further, they may be an average value of data measured this week and an average value of data measured last week, or may be an average value of data measured this month and an average value of data measured last month.

(Display Example 4)

Blood pressure values measured may be compared with each other and the high/low relation therebetween may be determined based on the comparison result. The determined result then may be displayed using a direction of an arrow 85, as shown in Figs. 14-16. Although the high/low relation is herein shown using the direction of an arrow, not limited to the arrow, any graphical pattern (symbol or picture) that can show the high/low relation with its direction may be used.

For example, in Fig. 14, an arrow 85 is displayed together with today's average data of SBP measured after getting up 64, average data of DBP measured after getting up 65 and average data of PLS measured after getting up 66. Risk value calculation and determination portion 24 compares today's average data 64-66 of SBP measured after getting up, DBP measured after getting up and PLS measured after getting up, with yesterday's average data of SBP measured before going to bed, DBP measured before going to bed and PLS measured before going to bed. Based on the comparison result, display control portion 25 displays whether the today's blood pressure is higher or lower than the yesterday's blood pressure using arrow 85. It is noted that the average values are obtained by average value calculation portion 23. Here, the result of comparison between average data of SBP measured after getting up 64 and the average data of SBP measured before going to bed shows a trend that average data of SBP measured after getting up 64 is higher than the average data of SBP measured before going to bed, so that arrow 85 is displayed upward in Fig. 14. If it is lower, arrow 85 will be displayed downward. If unchanged, arrow 85 will be displayed horizontally.

The comparison may be carried out using any one of the average data of systolic blood pressure, diastolic blood pressure and pulse rate after getting up and before going to bed, or using all of the average data of systolic blood pressure, diastolic blood pressure and pulse rate after getting up and before going to bed, or using either one of the average data of systolic blood pressure and diastolic blood pressure after getting up and before going to bed, or using both of the average data of systolic blood pressure and diastolic blood pressure after getting up and before going to bed. In the case where comparison is made using all the average data of systolic blood pressure, diastolic blood pressure and pulse rate, arrow 85 is displayed upward when at least one of those three types of data after getting up is higher than the corresponding one before going to bed, while arrow 85 is displayed downward if at least one of them is lower. Arrow 85 is displayed horizontally if there is no change in those three types of data. In the case where both of the systolic blood pressure and the diastolic blood pressure are compared to determine the trend, arrow 85 is displayed upward if at least one of the systolic blood pressure and the diastolic blood pressure after getting up is higher than the corresponding one before going to bed, and displayed downward if it is lower. It is displayed horizontally if there is no change between the two types of data.

Further, arrows 85 may be displayed corresponding to the respective average data 64-66 of SBP measured after getting up, DBP measured after getting up and PLS measured after getting up, as shown in Fig. 15. Today's average data 64-66 of SBP measured after getting up, DBP measured after getting up and PLS measured after getting up may be compared respectively with yesterday's average data of SBP measured before going to bed, DBP measured before going to bed and PLS measured before going to bed, and the directions of corresponding arrows 85 may be changed based on the comparison results.

It may also be possible to display, together with today's average data 64-66 of SBP measured after getting up, DBP measured after getting up and PLS measured after getting up, arrows 85 indicating the directions based on the comparison results of measurement data for this week, last week, and the week before last, as shown in Fig. 16. Specifically, risk value calculation and determination portion 24 performs comparison between today's average data of SBP measured after getting up 64 and this week's average data of SBP measured after getting up, comparison between today's average data of SBP measured after getting up 64 and last week's average data of SBP measured after getting up, and comparison between today's average data of SBP measured after getting up 64 and the week before last's average data of SBP measured after getting up. Display control portion 25 then displays arrows 85 in the directions based on the comparison results for the respective weeks. It is noted that the average values for the respective weeks are obtained by average value calculation portion 23.

In Figs. 14 and 15, comparison is made between today's average data 64-66 of SBP measured after getting up, DBP measured after getting up and PLS measured after getting up and yesterday's average data of SBP measured before going to bed, DBP measured before going to bed and PLS measured before going to bed. The targets of comparison however are not limited thereto. For example, comparison may be made between the current measurement value and the previous measurement value, between the measurement value after getting up and the measurement value before going to bed (on the basis of single measurement value or average value), between today's measurement value and yesterday's measurement value (on the basis of average value), between this week's measurement value and last week's measurement value (on the basis of average value), or between this month's measurement value and the last month's measurement value (on the basis of average value).

Further, although comparison is made between the measurement values in Fig. 14, comparison may be made between the measurement values and the reference values for use in risk determination (135 mmHg, 85 mmHg). Specifically, with today's average data 64-66 of SBP measured after getting up, DBP measured after getting up and PLS measured after getting up being displayed, risk value calculation and determination portion 24 determines whether the above-described prescribed relation is satisfied for today's average data of SBP measured after getting up 64 and average data of DBP measured after getting up 65 with respect to the reference values. If it is determined that the relation is satisfied, display control portion 25 displays arrow 85 upward. If it is determined that the relation is not satisfied, display control portion 25 displays arrow 85 downward.

(Display Example 5)

It may also be possible to compare the measured blood pressure values and determine the high/low relation therebetween based on the comparison result, and display the determined result using an icon 88 of a prescribed graphical pattern or pictorial symbol as shown in Figs. 17-19. Herein, the high/low relation is shown using the display manner of icon 88. The target data, the procedure of comparison and others in Figs. 17-19 are identical to those explained in conjunction with Figs. 14-16.

The display manner of icon 88 may be changed by means of presence/absence of display by turning on/off of the icon, blinking, change in display color, a combination of blinking and change in display color, or change in intervals of blinking.

Figs. 17-19 show the display examples when arrows 85 in Figs. 14-16 are replaced with icons 88. When the result of comparison between average data of SBP measured after getting up 64 and the average data of SBP measured before going to bed shows the trend that average data of SBP measured after getting up 64 is higher than the average data of SBP measured before going to bed, icon 88 is turned on in Fig. 17. If the same is lower, the icon remains blinking. If there is no change, the icon is turned off.

Further, icons 88 may be displayed corresponding to the respective average data 64-66 of SBP measured after getting up, DBP measured after getting up and PLS measured after getting up, as shown in Fig. 18. Today's average data 64-66 of SBP measured after getting up, DBP measured after getting up and PLS measured after getting up may be compared respectively with yesterday's average data of SBP measured before going to bed, DBP measured before going to bed and PLS measured before going to bed, and the display manners of corresponding icons 88 may be changed based on the comparison results.

Further, as shown in Fig. 19, icons 88 may be displayed based on the comparison results of measurement data for this week, last week, and the week before last, together with today's average data 64-66 of SBP measured after getting up, DBP measured after getting up and PLS measured after getting up. Risk value calculation and determination portion 24 performs comparison between today's average data of SBP measured after getting up 64 and this week's average data of SBP measured after getting up, comparison between today's average data of SBP measured after getting up 64 and last week's average data of SBP measured after getting up, and comparison between today's average data of SBP measured after getting up 64 and the week before last's average data of SBP measured after getting up, and display control portion 25 displays icons 88 corresponding to the respective weeks in the manners based on the comparison results.

(Display Example 6)

It may also be possible to compare the blood pressure measurement result data under different measurement conditions, and based on the comparison results, display the trends of high/low of the blood pressures using a bar-shaped indicator 86 as shown in Figs. 20-23.

Referring to Fig. 20, in display portion 4, indicator 86 is displayed together with today's average data 64-66 of SBP measured after getting up, DBP measured after getting up and PLS measured after getting up. Risk value calculation and determination portion 24 compares the value of average data of SBP measured after getting up 64 with the value of yesterday's average data of SBP measured before going to bed to obtain a difference therebetween, and display control portion 25 displays the obtained difference using indicator 86.

The display examples using indicator 86 are shown in Figs. 24A-24E. In the case where the difference obtained by subtracting the value of average data of SBP measured before going to bed from the value of average data of SBP measured after getting up exceeds -20 mmHg, indicator 86 is displayed as shown in Fig. 24A. Similarly, if the difference is from -10 mmHg to -20 mmHg, it is displayed as shown in Fig. 24B, and if the difference is ±10 mmHg, it is displayed as shown in Fig. 24C. If the difference is from 10 mmHg to 20 mmHg, it is displayed as shown in Fig. 24D, and if the difference exceeds 20 mmHg, it is displayed as shown in Fig. 24E. As such, with indicator 86 being displayed as a plurality of blocks stacked one on another in the bar shape, only one block indicating the corresponding level is displayed in reverse mode (filled in). This allows the user to recognize the difference by confirming the position of the block displayed in reverse mode within the bar-shaped indicator.

In Fig. 20, comparison is made between the average data of SBP measured after getting up and the average data of SBP measured before going to bed. The comparison targets however are not limited thereto. For example, the comparison may be carried out using any one of the average data of systolic blood pressure, diastolic blood pressure and pulse rate after getting up and before going to bed. It may be carried out using either one of the average data of systolic blood pressure and diastolic blood pressure after getting up and before going to bed. Further, it may be carried out using all the average data of systolic blood pressure, diastolic blood pressure and pulse rate after getting up and before going to bed, or it may be carried out using both of the average data of systolic blood pressure and diastolic blood pressure after getting up and before going to bed. In the case where comparison is made using a plurality of average data, it may be configured such that the largest one of the plurality of differences as the comparison results is displayed.

In Fig. 21, comparison is made between respective ones of today's average data 64-66 of SBP measured after getting up, DBP measured after getting up and PLS measured after getting up and respective ones of yesterday's average data of SBP measured before going to bed, DBP measured before going to bed and PLS measured before going to bed, and the corresponding indicators 86 are displayed based on the comparison results, in a similar manner as in Fig. 20.

In Fig. 22, together with today's average data 64-66 of SBP measured after getting up, DBP measured after getting up and PLS measured after getting up, indicators 86 indicating the trends based on the comparison results of measurement data are displayed for this week, last week, and the week before last. Comparison is made between average data of SBP measured after getting up 64 and this week's average data of SBP measured after getting up, between average data of SBP measured after getting up 64 and last week's average data of SBP measured after getting up, and between average data of SBP measured after getting up 64 and the week before last's average data of SBP measured after getting up, and based on the comparison results, the corresponding indicators 86 are displayed for the respective weeks, in a similar manner as in Fig. 20.

In Fig. 23, together with today's average data 64-66 of SBP measured after getting up, DBP measured after getting up and PLS measured after getting up, differences obtained by comparing today's average data of SBP measured after getting up 64 with respective average data of SBP measured after getting up for Monday through Sunday are displayed using indicators 86 prepared for the respective days of the week, in a similar manner as in Fig. 20.

In Figs. 20 and 21, comparison is made between today's average data 64-66 of SBP measured after getting up, DBP measured after getting up and PLS measured after getting up and yesterday's average data of SBP measured before going to bed, DBP measured before going to bed and PLS measured before going to bed. The targets of comparison however are not limited thereto. For example, comparison may be made between the current measurement value and the previous measurement value, between the measurement value after getting up and the measurement value before going to bed (on the basis of single measurement value or average value), between today's measurement value and yesterday's measurement value (on the basis of average value), between this week's measurement value and last week's measurement value (on the basis of average value), or between this month's measurement value and the last month's measurement value (on the basis of average value).

Further, although comparison is made between the measurement values in Fig. 20, comparison may be made between the measurement values and the risk determination reference values (135 mmHg, 85 mmHg). Specifically, with today's average data 64-66 of SBP measured after getting up, DBP measured after getting up and PLS measured after getting up being displayed, risk value calculation and determination portion 24 may obtain differences between today's average data of SBP measured after getting up 64 and average data of DBP measured after getting up 65 and the reference values (135 mmHg, 85 mmHg). Display control portion 25 may then display the obtained difference using indicator 86. The difference may be the difference between average data of SBP measured after getting up 64 and reference value of 135 mmHg, or the difference between average data of DBP measured after getting up 65 and reference value of 85 mmHg. The display example of such an indicator is shown in Fig. 25. In Fig. 25, the difference with reference value 80 is displayed using indicator 86.

(Display Example 7)

Other display examples of Figs. 20-25 are shown in Figs. 26-31. The displays of trends using indicators 86 in Figs. 20-25 may be modified to the displays as shown in Figs. 26-31. In Figs. 26-31, the difference between two measurement data under different measurement conditions as the comparison targets, or the difference between the measurement data and the reference value, is indicated by displaying at least one block out of a plurality of blocks constituting an indicator 87 in reverse mode (filled in). This allows the subject to recognize the amount of difference by confirming the total number of blocks displayed in reverse mode.

In the case of the display using the indicator of Display Example 7, it may be configured such that the indicator is displayed in reverse mode only when the value is higher than reference value 80.

(Display Example 8)

In Display Examples 6 and 7, the difference indicated by the comparison result has been displayed using indicators 86 and 87. In Display Example 8, the indicator is replaced with a LED 89. The measurement data being displayed, procedure of comparison and determination, and the data as the comparison targets are identical to those in Display Examples 6 and 7, except that indicator 86 or 87 is replaced with LED 89.

Display control portion 25 changes the display manner of LED 89 based on the comparison results between the blood pressure data as the targets, or based on the comparison results between the blood pressure data and reference values 80, as shown in Figs. 32-34. Specifically, the high/low relation shown by the comparison result is displayed by turning on or blinking of LED 89.

Alternatively, the amount of difference may be shown by lighting or blinking. For example, the LED may be turned off when the difference is less than 10 mmHg, it may be turned on when the difference is not less than 10 mmHg but less than 20 mmHg, and it may be made to blink when the difference is not less than 20 mmHg.

Further, the amount of difference may be shown by the color of emission of light of LED 89. For example, green, orange and red colors may be used to display the difference of less than 10 mmHg, the difference of not less than 10 mmHg and less than 20 mmHg, and the difference of not less than 20 mmHg, respectively, although the colors of emission of light are not limited thereto.

In Figs. 32-34, LED 89 is provided as a part of display portion 4. Alternatively, it may be provided external to display portion 4 in Fig. 2, in the vicinity of display portion 4 so as to be associated with the displayed data.

In Display Examples 4-8 described above, average value calculation portion 23 reads the blood pressure measurement data from areas 26 and 27 of memory 12 shown in Fig. 3A, and calculates the averages of the read data, as described above. Display control portion 25 then selects and displays the average data calculated for the blood pressures measured under any one measurement condition. Thus, the average data being displayed may be average data for area 26, or average data for area 27. The instruction as to which average data is to be selected may be provided by the user via manipulation portion 40, or it may be predetermined.

(Display Example 9)

The trend of high/low of blood pressure based on the comparison results between the blood pressure data or based on the comparison results between the blood pressure data and reference values 80, as described above, may be notified to the subject by vibration of blood pressure monitor main body 2 by a vibration portion 48 or by a sound output by a sound output portion 47, together with the display on display portion 4.

Specifically, CPU 20 controls vibration portion 48 or sound output portion 47 based on the determined trend of high/low. With this control, the trend of high or low may be indicated by difference in vibration intervals or vibration pattern of vibration portion 48. Similarly, it may be indicated by difference in tone, scale or rhythm of the sound output from sound output portion 47. It is noted that the trend of high or low may be indicated using only sound output portion 47 or vibration portion 48, without the display on display portion 4.

(Display Example 10)

Another display example will be described with reference to Figs. 35A and 35B.

Display portion 4 in Figs. 35A and 35B has display areas 4A, 4B and 4C. In display area 4A, today’s average data 64-66 of SBP measured after getting up, DBP measured after getting up and PLS measured after getting up are displayed. In display area 4C, a morning mark 91 (picture, symbol, letters and the like) is displayed to indicate that the data being displayed on display area 4A is the blood pressure values measured after getting up (in the morning). In Figs. 35A and 35B, the letters of "MORNING" are displayed as mark 91.

Risk value calculation and determination portion 24 compares the value indicated by the blood pressure data in display area 4A with the risk determination reference value (135 mmHg, 85 mmHg), and outputs the comparison result. When the blood pressure data in display area 4A is higher (or exceeds) the reference value, display control portion 25 changes the display manner in display portion 4 based on the output comparison result. For example, it changes the display manner of data in display area 4A, or lights (displays) in display area 4B an icon 90 or other symbol or picture indicating that the data exceeds the reference value, or carries out the both. The display manner of data in display area 4A may be changed by causing the backlight to blink, by changing the color of emission of light, or by turning on (or off) the backlight. The data for displaying icon 90 and the morning mark in display areas 4B and 4C are pre-stored in a prescribed area in memory 12. Display control portion 25 reads the relevant data from memory 12 and displays icon 90 and morning mark 91 based thereon.

It may be possible to change the display manner of mark 91 in display area 4C instead of changing the display manner of data in display area 4A. For example, mark 91 may be made to start blinking, or the display color may be changed.

In the case where the blood pressure data in display area 4A exceeds the reference value, change in display manner of the data in display area 4A, lighting of icon 90 in display area 4B, and change in display manner of mark 91 in display area 4C may all be carried out.

Meanwhile, if the blood pressure data in display area 4A is not higher than (or does not exceed) the reference value, the display manner in each display area is not changed, as shown in Fig. 35B.

Although the present display example uses the average blood pressure values measured after getting up, the average blood pressure values measured before going to bed may be employed in a similar manner. Further, the average blood pressure values over a prescribed time period, for example, one day, one week, one month, one year and the like, may also be employed in a similar manner.

As described above, in Display Example 10, the average blood pressure data is displayed on display portion 4 as the representative data of blood pressure data group measured either after getting up (in the morning) or before going to bed (in the evening). In addition thereto, as the relation between the average blood pressure value being displayed and the reference value, the high/low relation shown by the comparison result therebetween is displayed by a particular display manner of the average blood pressure data being displayed, or by presence/absence of lighting of icon 90, or by a particular display manner of morning (evening) mark 91.

Further, a bar-shaped indicator 87 as described above may be displayed on display portion 4 to indicate the difference between the reference value and the average blood pressure value in display area 4A based on the comparison result.

(Display Example 11)

In the present display example, risk value calculation and determination portion 24 compares yesterday's average data 641, 651 and 661 of SBP measured before going to bed, DBP measured before going to bed and PLS measured before going to bed, calculated by average value calculation portion 23, and this morning's average data of SBP measured after getting up, DBP measured after getting up and PLS measured after getting up, calculated by average value calculation portion 23, with the risk determination reference values. Display control portion 25 then changes the display manner of data in display portion 4 based on the comparison results.

The display examples will now be described with reference to Figs. 36A-36C.

Display portion 4 in Figs. 36A-36C has display areas 4A, 4B and 4C. Display control portion 25 displays in display area 4A yesterday's average data 641, 651 and 661 of SBP measured before going to bed, DBP measured before going to bed and PLS measured before going to bed, and displays in display area 4C an evening mark (picture, symbol, letters and the like) 91 indicating that the data displayed in display area 4A are the blood pressure values measured before going to bed (in the evening). Display area 4B is provided for displaying icon 90. Risk value calculation and determination portion 24 compares the value indicated by the average data of SBP measured after getting up and DBP measured after getting up calculated by average value calculation portion 23 with the reference values. If it is determined that they exceed the reference values, icon 90 is lighted (displayed) in display area 4B by display control portion 25.

Fig. 36A shows the display example in the case where it is determined, as a result of comparison of the blood pressure values indicated by average data 641 and 651 of SBP measured before going to bed and DBP measured before going to bed with the reference values, that the average blood pressure values exceed the reference values. Specifically, display control portion 25 changes the display manner of the data in display area 4A. Although the backlight is herein made to blink by way of example, the color may be changed, or the backlight may be turned on (or off). Further, if it is determined that the average blood pressure values after getting up also exceed the reference values as a result of comparison of the blood pressure values indicated by the average data of SBP measured after getting up and DBP measured after getting up with the reference values, display control portion 25 lights icon 90 in display area 4B. It is noted that the display manner of evening mark 91 in display area 4C may be changed (e.g., it may be made to blink or the display color may be changed) instead of changing the display manner of the data in display area 4A. Alternatively, the display manner may be changed in both display areas 4A and 4C.

Fig. 36B shows the display example in the case where it is determined, as a result of comparison between the blood pressure values indicated by average data 641 and 651 of SBP measured before going to bed and DBP measured before going to bed and the reference values, that the average blood pressure values do not exceed (or are not greater than) the reference values. Specifically, display control portion 25 does not change the display manner of data in display area 4A. Meanwhile, since it is determined that the blood pressure values indicated by the average data of SBP measured after getting up and DBP measured after getting up exceed the reference values as a result of comparison therebetween, display control portion 25 lights icon 90 in display area 4B.

Fig. 36C shows the display example in the case where it is determined, as a result of comparison between the blood pressure values indicated by average data 641 and 651 of SBP measured before going to bed and DBP measured before going to bed and the reference values, that the average blood pressure values do not exceed (or are not greater than) the reference values. Specifically, display control portion 25 does not change the display manner of data in display area 4A. Further, since it is determined, as a result of comparison between the blood pressure values indicated by the average data of SBP measured after getting up and DBP measured after getting up and the reference values, that the average blood pressure values after getting up are not greater than (or do not exceed) the reference values as well, display control portion 25 does not light icon 90 in display area 4B.

Herein, of the average data measured before going to bed and measured after getting up, those measured before going to bed are displayed in display area 4A. Instead, the average data measured after getting up may be displayed. In such a case, icon 90 is lighted in display area 4B based on the comparison results between the values of average data measured before going to bed and the reference values. Further, morning mark 91 as in Figs. 35A and 35B is displayed in display area 4C.

In Display Examples 10 and 11 above, the representative blood pressure value of the blood pressure data group measured under a prescribed condition is displayed in display area 4A, and in the case where the representative blood pressure value exceeds a reference value for use in risk determination, the display manner of the blood pressure value in display area 4A or/and the display manner of the data indicating the measurement condition (morning or evening mark 91 in display area 4C) are set to a prescribed manner, or/and a particular symbol (icon 90) is displayed in display area 4B. It is noted that in place of or in addition to lighting of the particular symbol (icon 90), a lamp (not shown) may be made to turn on or blink, or a sound like a buzzer or voice may be output from sound output portion 47, or vibration may be caused via vibration portion 48.

Further, a difference between the average blood pressure value in display area 4A and the reference value based on the comparison result may be displayed in display portion 4 using a bar-shaped indicator 87 as described above.

As described above, in Display Example 11, the average blood pressure data serving as the representative data of the blood pressure data group measured either after getting up (in the morning) or before going to bed (in the evening) is displayed in display portion 4, and additionally, the relation between the average blood pressure values in the respective blood pressure data groups and the reference values is shown, as the high/low relation indicated by the comparison results therebetween, by a particular display manner of the average blood pressure data being displayed, by presence/absence of lighting of icon 90, or by a particular display manner of the morning (evening) mark.

Although the present invention has been described and illustrated in detail, it is clearly understood that the same is by way of illustration and example only and is not to be taken by way of limitation, the spirit and scope of the present invention being limited only by the terms of the appended claims.

## Claims

**1.** An electronic blood pressure monitor (1), comprising:

a blood pressure measurement unit measuring a blood pressure of a subject;
a memory unit (12);
a display unit (4);
a data storing unit (30) storing blood pressure data indicating said blood pressure measured by said blood pressure measurement unit in said memory unit in association with a type of a measurement condition at the time of blood pressure measurement; and
a control unit (20);
said control unit being configured to
read a blood pressure data group measured under said measurement condition of a first type and a blood pressure data group measured under said measurement condition of a second type different from said first type from said memory unit,
display one of representative blood pressure data representing the read blood pressure data group measured under said measurement condition of said first type and representative blood pressure data representing the read blood pressure data group measured under said measurement condition of said second type on said display unit, and
further display a relation between the read blood pressure data group measured under said measurement condition of said first type and the read blood pressure data group measured under said measurement condition of said second type, on said display unit in a prescribed display manner,
said relation including a high/low relation between a blood pressure indicated by said representative blood pressure data representing the blood pressure data group measured under said measurement condition of said first type and a blood pressure indicated by said representative blood pressure data representing the blood pressure data group measured under said measurement condition of said second type.

**2.** The electronic blood pressure monitor according to claim 1, wherein said control unit includes a calculation unit (23) calculating said representative blood pressure data based on said blood pressure data included in said blood pressure data group.

3. The electronic blood pressure monitor according to claim 2, wherein said calculation unit calculates an average of said blood pressure data included in said blood pressure data group as said representative blood pressure data.

4. The electronic blood pressure monitor according to claim 1, wherein said prescribed display manner indicates a manner of displaying a difference between said blood pressures having said high/low relation using a bar-shaped indicator.

5. The electronic blood pressure monitor according to claim 1, wherein said prescribed display manner indicates a manner of changing a display color of said representative blood pressure data displayed on said display unit in accordance with said high/low relation.

6. The electronic blood pressure monitor according to claim 1, wherein said prescribed display manner indicates a manner of displaying said representative blood pressure data on said display unit with presence/absence of blinking in accordance with said high/low relation.

7. The electronic blood pressure monitor according to claim 1, wherein said prescribed display manner corresponds to a manner of displaying or not displaying a prescribed graphical pattern in accordance with said high/low relation.

8. The electronic blood pressure monitor according to claim 1, wherein said prescribed display manner indicates a manner of causing a backlight of said representative blood pressure data in said display unit to be turned on or off, to be changed in color of emission of light, or to be made to blink, in accordance with said high/low relation.

9. The electronic blood pressure monitor according to claim 1, wherein said control unit notifies of said high/low relation with vibration or sound.

10. The electronic blood pressure monitor according to claim 1, wherein said measurement condition of said first type indicates measurement in the morning, and said measurement condition of said second type indicates measurement in the evening.

11. The electronic blood pressure monitor according to claim 1, wherein said measurement condition of said first type indicates measurement within a prescribed period after getting up, and said measurement condition of said second type indicates measurement within a prescribed period before going to bed.

12. The electronic blood pressure monitor according to claim 1, wherein said measurement condition of said second type indicates measurement made at least one day before a time indicated by said measurement condition of said first type.

13. An electronic blood pressure monitor, comprising:

a blood pressure measurement unit measuring a blood pressure of a subject;
a memory unit (12);
a display unit (4);
a data storing unit (30) storing blood pressure data indicating said blood pressure measured by said blood pressure measurement unit in said memory unit in association with a type of a measurement condition at the time of blood pressure measurement; and
a control unit (20);
said control unit being configured to
read one of a blood pressure data group measured under said measurement condition of a first type and a blood pressure data group measured under said measurement condition of a second type different from said first type from said memory unit,
display representative blood pressure data representing the read blood pressure data group on said display unit, and
further display a relation between the read blood pressure data group and reference blood pressure data indicating a reference blood pressure for use in blood pressure evaluation, on said display unit in a prescribed display manner,
said relation including a high/low relation between a blood pressure indicated by said representative blood pressure data of the read blood pressure data group and said reference blood pressure indicated by said reference blood pressure data.

**14.** An electronic blood pressure monitor (1), comprising:

a blood pressure measurement unit measuring a blood pressure of a subject;
a memory unit (12);
a display unit (4);
a data storing unit (30) storing blood pressure data indicating said blood pressure measured by said blood pressure measurement unit in said memory unit in association with a type of a measurement condition at the time of blood pressure measurement; and
a control unit (20);
said control unit being configured to
read both of a blood pressure data group measured under said measurement condition of a first type and a blood pressure data group measured under said measurement condition of a second type different from said first type from said memory unit,
display representative blood pressure data representing one of the read blood pressure data groups on said display unit, and
further display a relation between representative blood pressure data representing the other of the read blood pressure data groups and reference blood pressure data indicating a reference blood pressure for use in blood pressure evaluation, on said display unit in a prescribed display manner,
said relation including a high/low relation between a blood pressure indicated by said representative blood pressure data and said reference blood pressure indicated by said reference blood pressure data.

**15.** An electronic blood pressure monitor (1), comprising:

a blood pressure measurement unit measuring a blood pressure of a subject;
a memory unit (12);
a display unit (4);
a data storing unit (30) storing blood pressure data indicating said blood pressure measured by said blood pressure measurement unit in said memory unit in association with a type of a measurement condition at the time of blood pressure measurement; and
a control unit (20);
said control unit being configured to
read both of a blood pressure data group measured under said measurement condition of a first type and a blood pressure data group measured under said measurement condition of a second type different from said first type from said memory unit,
display representative blood pressure data representing one of the read blood pressure data groups on said display unit, and
further display, for each of the read blood pressure data groups, a relation between representative blood pressure data representing the blood pressure data group and reference blood pressure data indicating a reference blood pressure for use in blood pressure evaluation, separately on said display unit in a prescribed display manner,
said relation including a high/low relation between a blood pressure indicated by said representative blood pressure data and said reference blood pressure indicated by said reference blood pressure data.

**16.** An electronic blood pressure monitor (1), comprising:

a blood pressure measurement unit measuring a blood pressure of a subject;
a memory unit (12);
a display unit (4);
a data storing unit (30) storing data of said blood pressure measured by said blood pressure measurement unit in said memory unit in association with a type of a measurement condition at the time of blood pressure measurement; and
a control unit (20);
said control unit being configured to
read a blood pressure data group measured under said measurement condition of a first type and a blood pressure data group measured under said measurement condition of a second type different from said first type from said memory unit, and
display representative blood pressure data representing the read blood pressure data group measured under said measurement condition of said first type and representative blood pressure data representing the read blood pressure data group measured under said measurement condition of said second type, on said display unit at the same time.

**17.** A blood pressure measurement data processing apparatus (31), comprising:

a memory unit (33) having data of a measured blood pressure stored in association with a type of a measurement condition at the time of blood pressure measurement;
a display unit (34); and
a data processing unit (32);
said data processing unit being configured to
read a blood pressure data group measured under said measurement condition of a first type and a blood pressure data group measured under said measurement condition of a second type different from said first type from said memory unit,
display one of representative blood pressure data representing the read blood pressure data group measured under said measurement condition of said first type and representative blood pressure data representing the read blood pressure data group measured under said measurement condition of said second type on said display unit, and
further display a relation between the read blood pressure data group measured under said measurement condition of said first type and the read blood pressure data group measured under said measurement condition of said second type, on said display unit in a prescribed display manner.

**18.** A blood pressure measurement data processing apparatus (31), comprising:

a memory unit (33) having data of a measured blood pressure stored in association with a type of a measurement condition at the time of blood pressure measurement;
a display unit (34); and
a data processing unit (32);
said data processing unit being configured to
read one of a blood pressure data group measured under said measurement condition of a first type and a blood pressure data group measured under said measurement condition of a second type different from said first type from said memory unit,
display representative blood pressure data representing the read blood pressure data group on said display unit, and
further display a relation between the read blood pressure data group and reference blood pressure data for use in blood pressure evaluation, on said display unit in a prescribed display manner.

**19.** A blood pressure measurement data processing apparatus (31), comprising:

a memory unit (33) having data of a measured blood pressure stored in association with a type of a measurement condition at the time of blood pressure measurement;
a display unit (34); and
a data processing unit (32);
said data processing unit being configured to
read both of a blood pressure data group measured under said measurement condition of a first type and a blood pressure data group measured under said measurement condition of a second type different from said first type from said memory unit,
display representative blood pressure data representing one of the read blood pressure data groups, and
further display a relation between representative blood pressure data representing the other of the read blood pressure data groups and reference blood pressure data indicating a reference blood pressure for use in blood pressure evaluation, on said display unit in a prescribed display manner.

**20.** A blood pressure measurement data processing apparatus (31), comprising:

a memory unit (33) having data of a measured blood pressure stored in association with a type of a measurement condition at the time of blood pressure measurement;
a display unit (34); and
a data processing unit (32);
said data processing unit being configured to
read both of a blood pressure data group measured under said measurement condition of a first type and a blood pressure data group measured under said measurement condition of a second type different from said first type from said memory unit,
display representative blood pressure data representing one of the read blood pressure data groups on said display unit, and
further display, for each of the read blood pressure data groups, a relation between representative blood pressure data representing the blood pressure data group and reference blood pressure data indicating a reference blood pressure for use in blood pressure evaluation, separately on said display unit in a prescribed display manner.

**21.** A blood pressure measurement data processing method using a computer having a memory (12) and a data processing unit (20), comprising:

a storing step of storing data of a measured blood pressure in said memory in association with a type of a measurement condition at the time of blood pressure measurement; and
a data processing step executed by said data processing unit;
said data processing step including the steps of
reading a blood pressure data group measured under said measurement condition of a first type and a blood pressure data group measured under said measurement condition of a second type different from said first type from said memory,
displaying one of representative blood pressure data representing the read blood pressure data group measured under said measurement condition of said first type and representative blood pressure data representing the read blood pressure data group measured under said measurement condition of said second type, and
further displaying a relation between the read blood pressure data group measured under said measurement condition of said first type and the read blood pressure data group measured under said measurement condition of said second type, in a prescribed display manner.

**22.** A blood pressure measurement data processing method using a computer having a memory (12) and a data processing unit (20), comprising:

a storing step of storing data of a measured blood pressure in said memory in association with a type of a measurement condition at the time of blood pressure measurement; and
a data processing step executed by said data processing unit;
said data processing step including the steps of
reading one of a blood pressure data group measured under said measurement condition of a first type and a blood pressure data group measured under said measurement condition of a second type different from said first type from said memory,
displaying representative blood pressure data representing the read blood pressure data group, and
further displaying a relation between the read blood pressure data group and reference blood pressure data for use in blood pressure evaluation, in a prescribed display manner.

**23.** A blood pressure measurement data processing method using a computer having a memory (12) and a data processing unit (20), comprising:

a storing step of storing data of a measured blood pressure in said memory in association with a type of a measurement condition at the time of blood pressure measurement; and
a data processing step executed by said data processing unit;
said data processing step including the steps of
reading both of a blood pressure data group measured under said measurement condition of a first type and a blood pressure data group measured under said measurement condition of a second type different from said first type from said memory,
displaying representative blood pressure data representing one of the read blood pressure data groups, and
further displaying a relation between representative blood pressure data representing the other of the read blood pressure data groups and reference blood pressure data indicating a reference blood pressure for use in blood pressure evaluation, in a prescribed display manner.

**24.** A blood pressure measurement data processing method using a computer having a memory (12) and a data processing unit (20), comprising:

a storing step of storing data of a measured blood pressure in said memory in association with a type of a measurement condition at the time of blood pressure measurement; and
a data processing step executed by said data processing unit;
said data processing step including the steps of
reading both of a blood pressure data group measured under said measurement condition of a first type and a blood pressure data group measured under said measurement condition of a second type different from said first type from said memory,
displaying representative blood pressure data representing one of the read blood pressure data groups, and further displaying, for each of the read blood pressure data groups, a relation between representative blood pressure data representing the blood pressure data group and reference blood pressure data indicating a reference blood pressure for use in blood pressure evaluation, separately in a prescribed display manner.

**25.** A computer-readable recording medium recording a program for causing a computer to execute the blood pressure measurement data processing method recited in claim 21.

**26.** A computer-readable recording medium recording a program for causing a computer to execute the blood pressure measurement data processing method recited in claim 22.

**27.** A computer-readable recording medium recording a program for causing a computer to execute the blood pressure measurement data processing method recited in claim 23.

**28.** A computer-readable recording medium recording a program for causing a computer to execute the blood pressure measurement data processing method recited in claim 24.

**29.** A program for causing a computer to execute the blood pressure measurement data processing method recited in claim 21.

**30.** A program for causing a computer to execute the blood pressure measurement data processing method recited in claim 22.

**31.** A program for causing a computer to execute the blood pressure measurement data processing method recited in claim 23.

**32.** A program for causing a computer to execute the blood pressure measurement data processing method recited in claim 24.

FIG.1

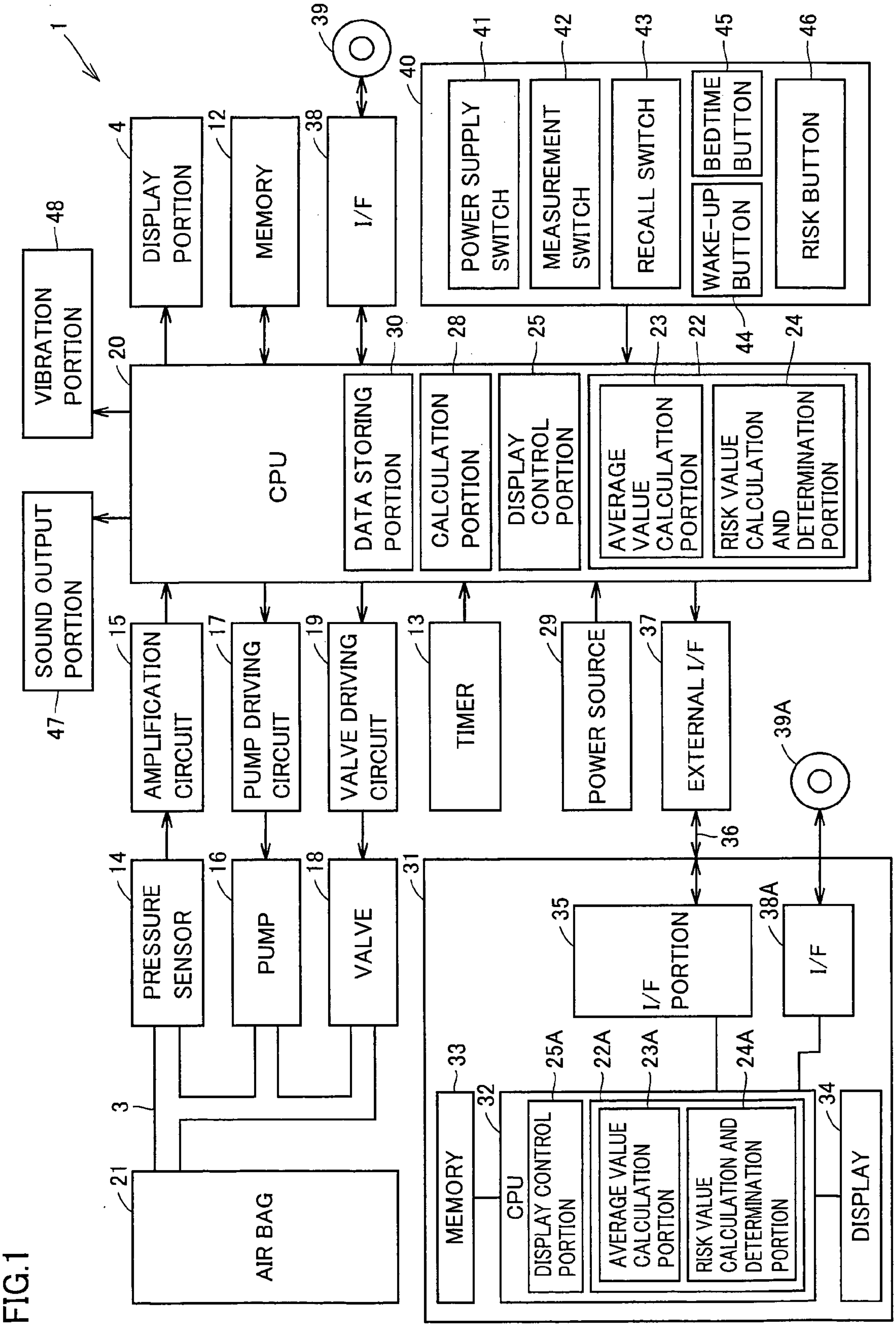
1
VIBRATION PORTION
48
SOUND OUTPUT PORTION
47
DISPLAY PORTION
4
MEMORY
12
I/F
38
39
CPU
20
DATA STORING PORTION
30
CALCULATION PORTION
28
DISPLAY CONTROL PORTION
25
AVERAGE VALUE CALCULATION PORTION
23
22
RISK VALUE CALCULATION AND DETERMINATION PORTION
24
40
POWER SUPPLY SWITCH
41
MEASUREMENT SWITCH
42
RECALL SWITCH
43
BEDTIME BUTTON
45
WAKE-UP BUTTON
44
RISK BUTTON
46
AMPLIFICATION CIRCUIT
15
PUMP DRIVING CIRCUIT
17
VALVE DRIVING CIRCUIT
19
TIMER
13
POWER SOURCE
29
EXTERNAL I/F
37
39A
36
PRESSURE SENSOR
14
PUMP
16
VALVE
18
3
AIR BAG
21
31
I/F PORTION
35
I/F
38A
MEMORY
33
CPU
32
DISPLAY CONTROL PORTION
25A
22A
AVERAGE VALUE CALCULATION PORTION
23A
RISK VALUE CALCULATION AND DETERMINATION PORTION
24A
DISPLAY
34

FIG.2

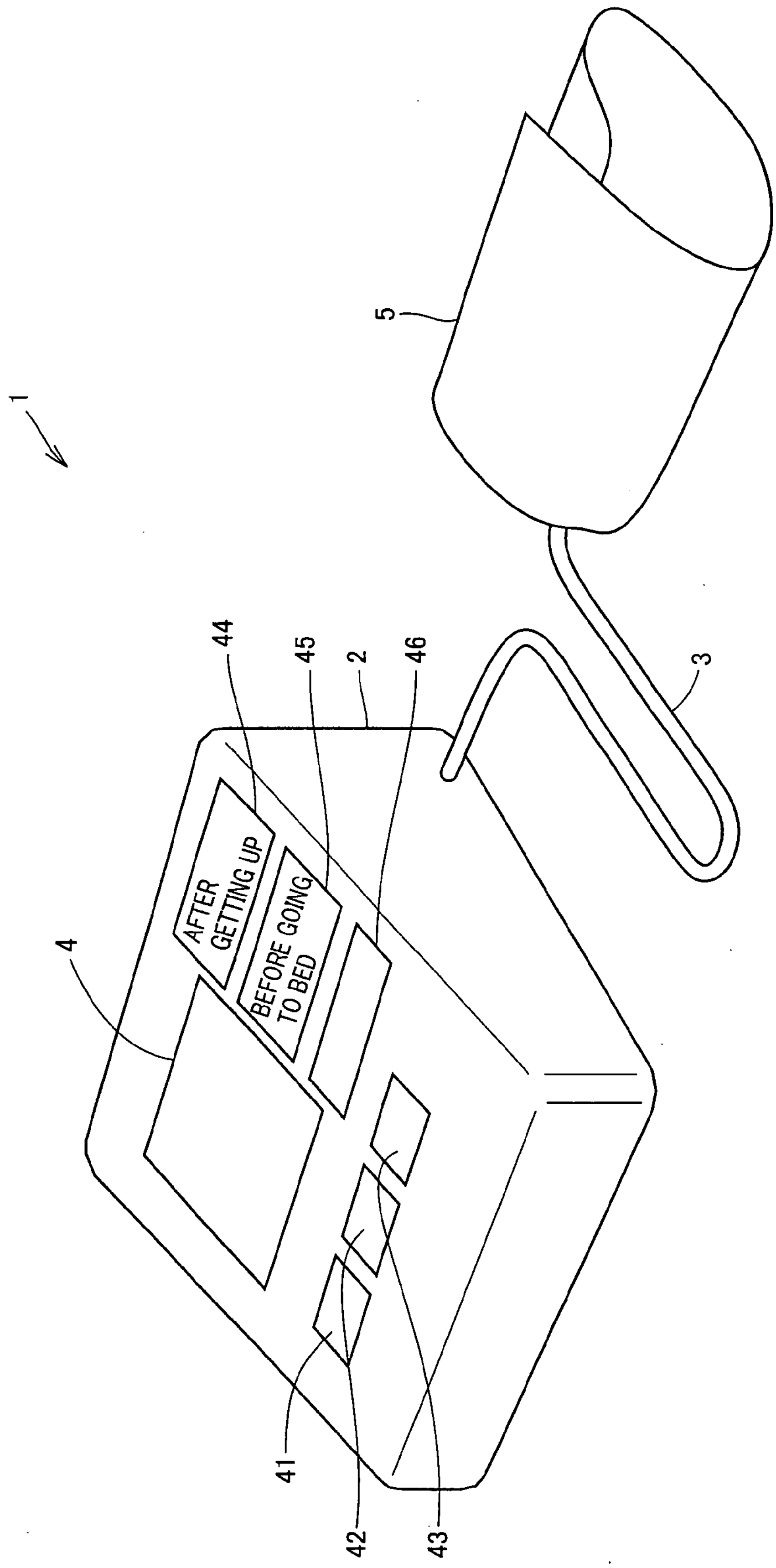

FIG.3B

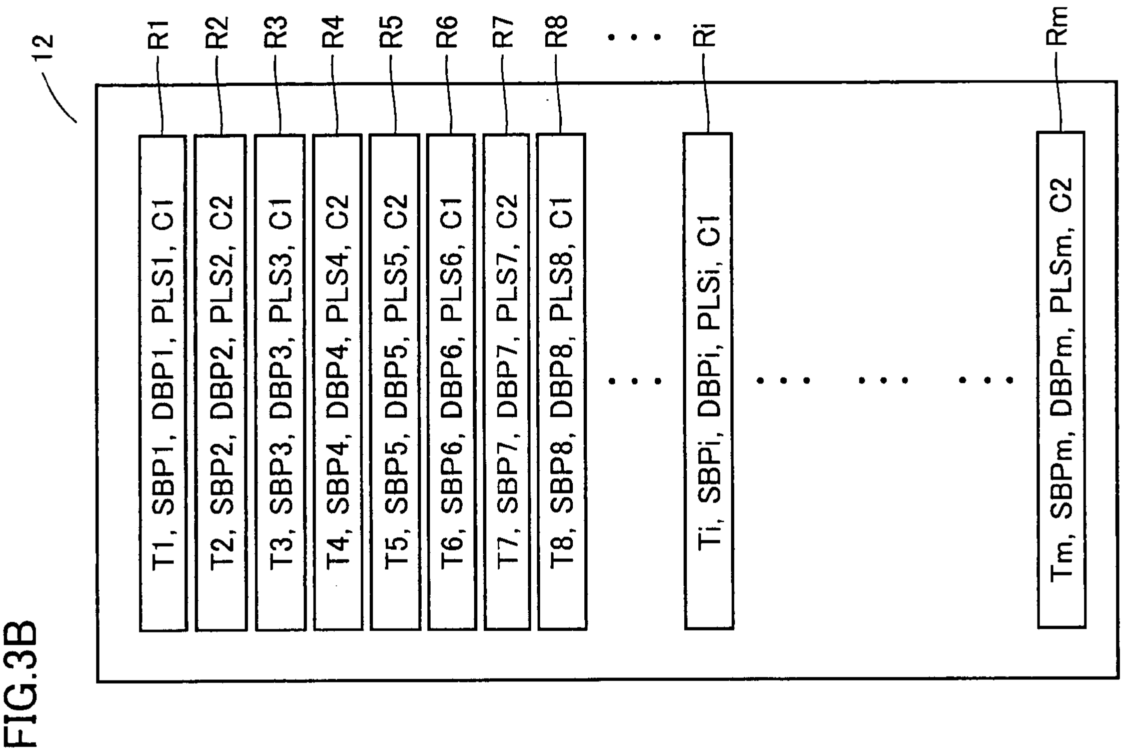
12
R1
R2
R3
R4
R5
R6
R7
R8
Ri
Rm
T1, SBP1, DBP1, PLS1, C1
T2, SBP2, DBP2, PLS2, C2
T3, SBP3, DBP3, PLS3, C1
T4, SBP4, DBP4, PLS4, C2
T5, SBP5, DBP5, PLS5, C2
T6, SBP6, DBP6, PLS6, C1
T7, SBP7, DBP7, PLS7, C2
T8, SBP8, DBP8, PLS8, C1
Ti, SBPi, DBPi, PLSi, C1
Tm, SBPm, DBPm, PLSm, C2

FIG.3A

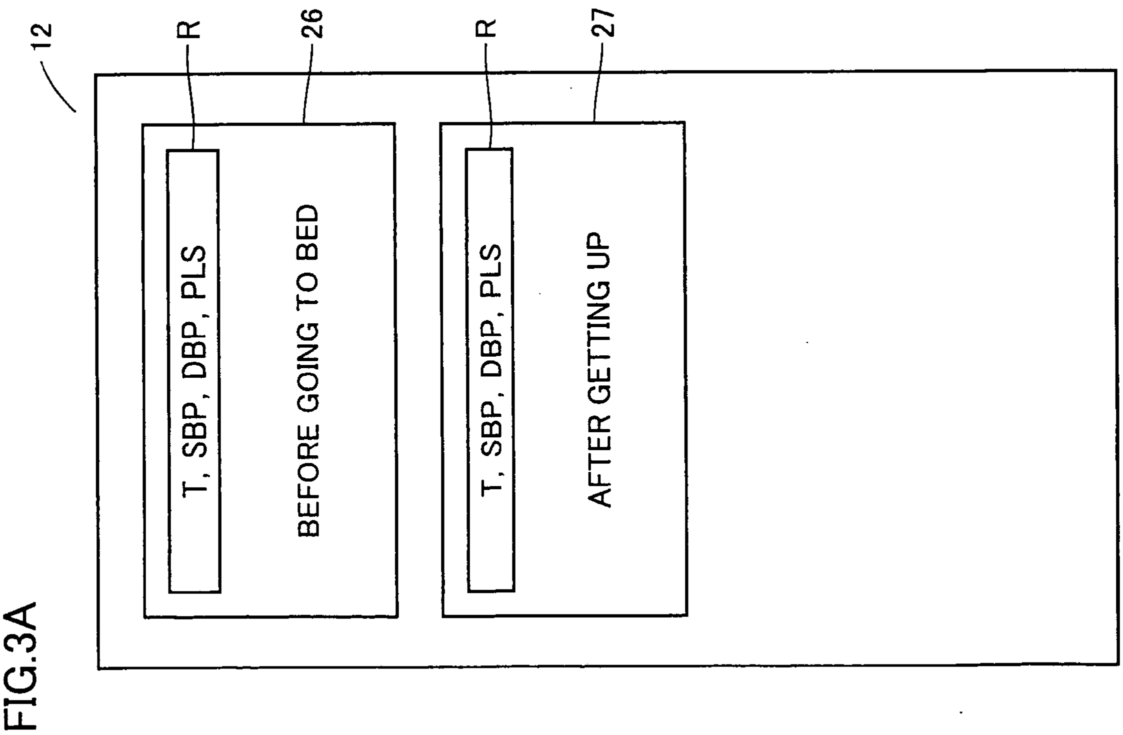
12
R
26
R
27
T, SBP, DBP, PLS
BEFORE GOING TO BED
T, SBP, DBP, PLS
AFTER GETTING UP

FIG.4

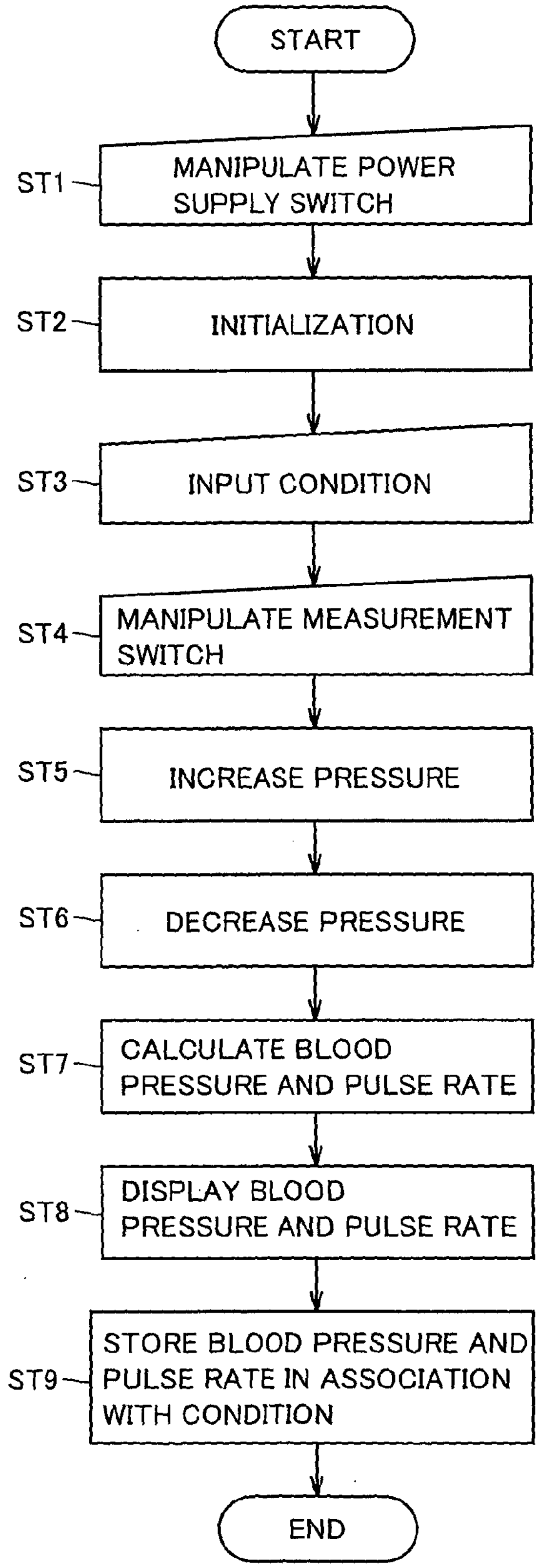
START
ST1
MANIPULATE POWER SUPPLY SWITCH
ST2
INITIALIZATION
ST3
INPUT CONDITION
ST4
MANIPULATE MEASUREMENT SWITCH
ST5
INCREASE PRESSURE
ST6
DECREASE PRESSURE
ST7
CALCULATE BLOOD PRESSURE AND PULSE RATE
ST8
DISPLAY BLOOD PRESSURE AND PULSE RATE
ST9
STORE BLOOD PRESSURE AND PULSE RATE IN ASSOCIATION WITH CONDITION
END

FIG.5

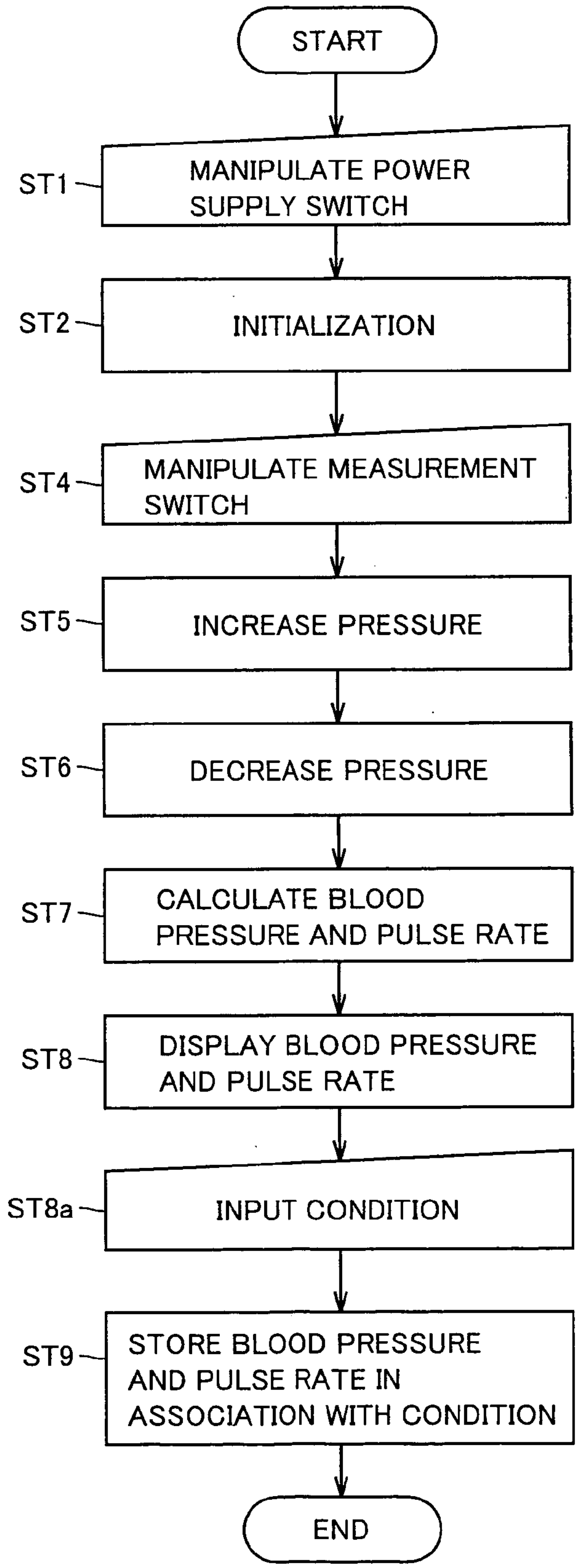
START
ST1
MANIPULATE POWER SUPPLY SWITCH
ST2
INITIALIZATION
ST4
MANIPULATE MEASUREMENT SWITCH
ST5
INCREASE PRESSURE
ST6
DECREASE PRESSURE
ST7
CALCULATE BLOOD PRESSURE AND PULSE RATE
ST8
DISPLAY BLOOD PRESSURE AND PULSE RATE
ST8a
INPUT CONDITION
ST9
STORE BLOOD PRESSURE AND PULSE RATE IN ASSOCIATION WITH CONDITION
END

FIG.6

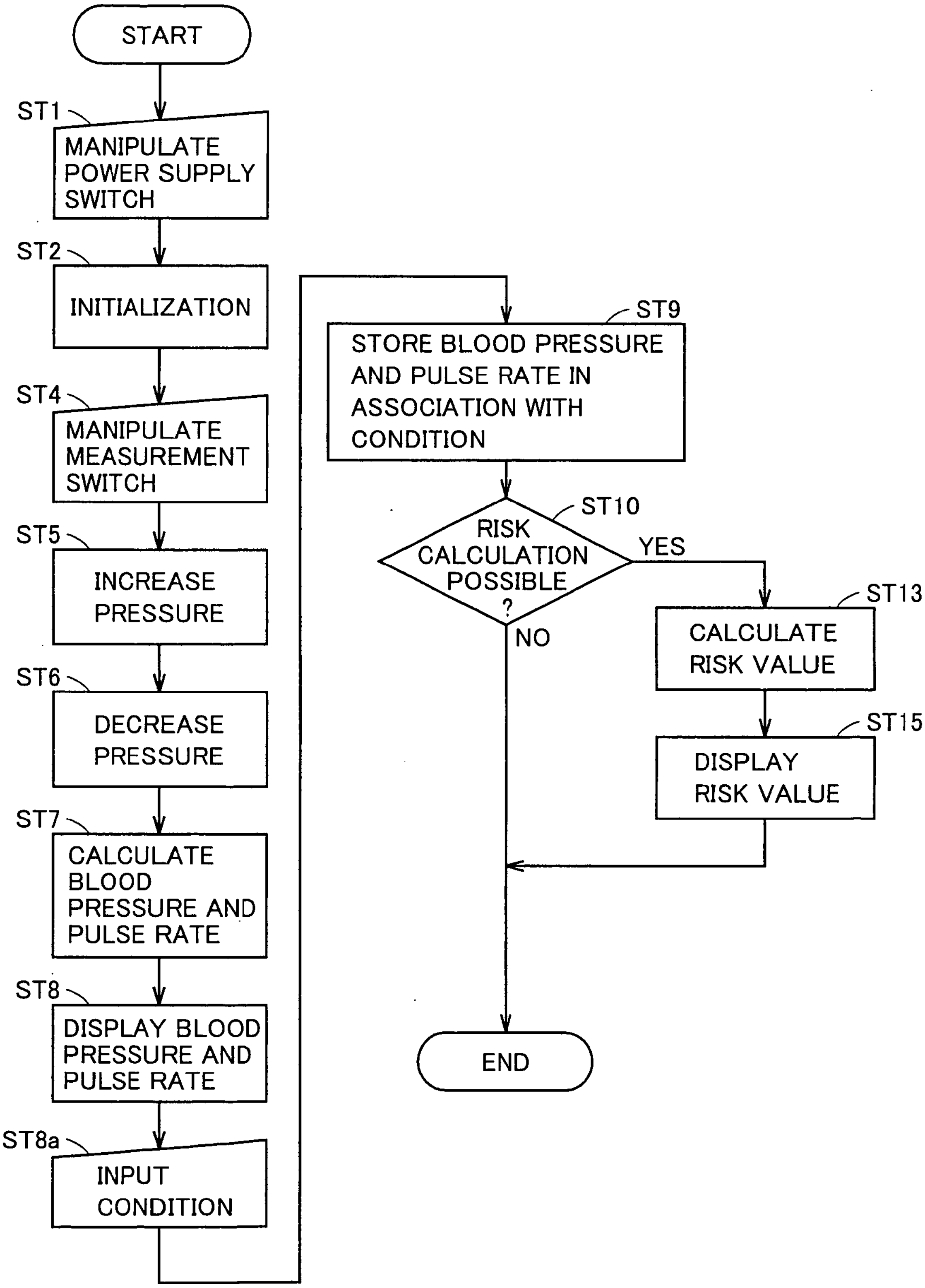
START
ST1
MANIPULATE POWER SUPPLY SWITCH
ST2
INITIALIZATION
ST4
MANIPULATE MEASUREMENT SWITCH
ST5
INCREASE PRESSURE
ST6
DECREASE PRESSURE
ST7
CALCULATE BLOOD PRESSURE AND PULSE RATE
ST8
DISPLAY BLOOD PRESSURE AND PULSE RATE
ST8a
INPUT CONDITION
ST9
STORE BLOOD PRESSURE AND PULSE RATE IN ASSOCIATION WITH CONDITION
ST10
RISK CALCULATION POSSIBLE ?
YES
NO
ST13
CALCULATE RISK VALUE
ST15
DISPLAY RISK VALUE
END

FIG.7

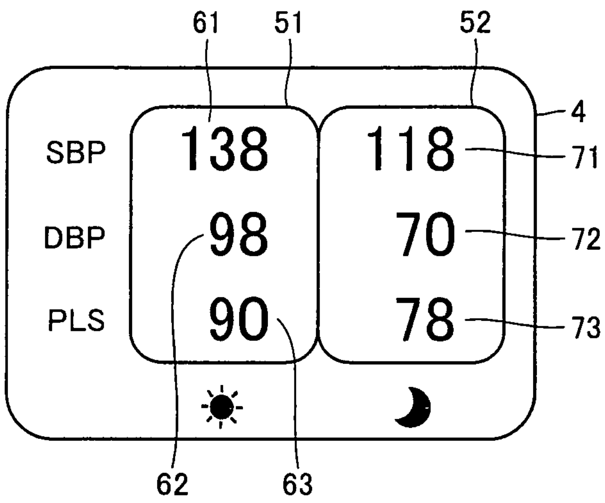
61
51
52
4
SBP
DBP
PLS
138
98
90
118
70
78
71
72
73
62
63

FIG.8

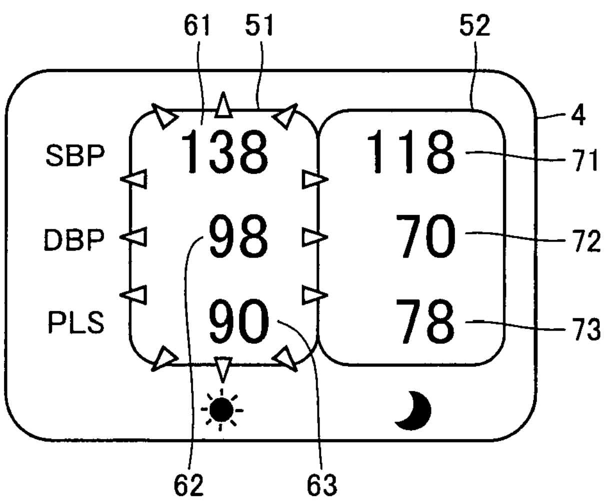
61
51
52
4
SBP
DBP
PLS
138
98
90
118
70
78
71
72
73
62
63

FIG.9

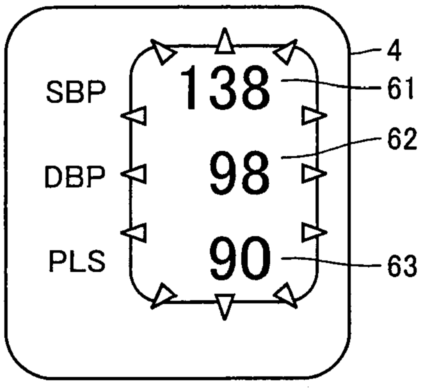
SBP
DBP
PLS
138
98
90
4
61
62
63

FIG.10

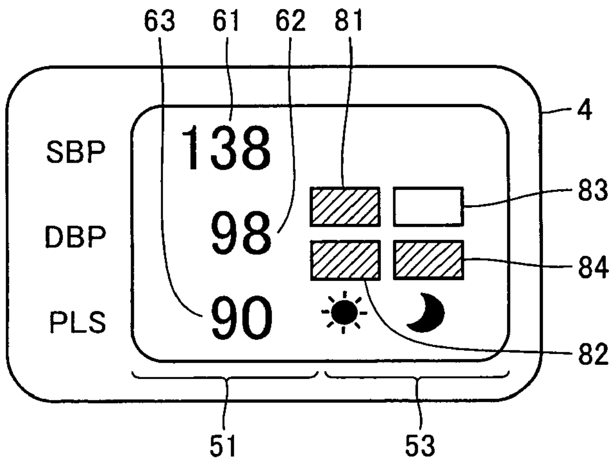
63
61
62
81
SBP
DBP
PLS
138
98
90
4
83
84
82
51
53

FIG.11A

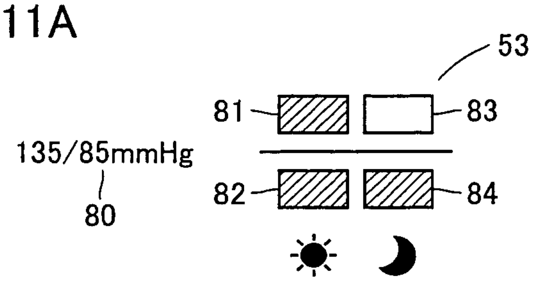


FIG.11B

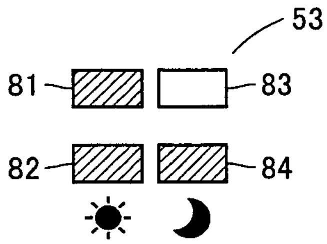


FIG.11C

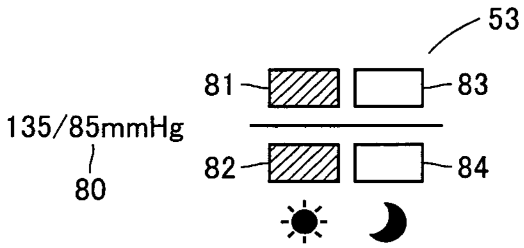


FIG.11D

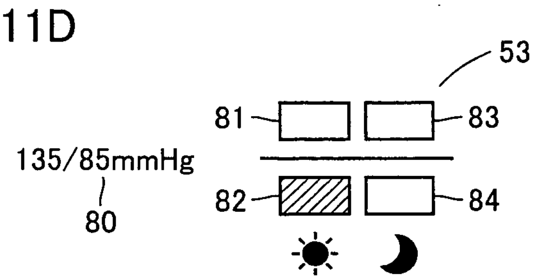

FIG.12

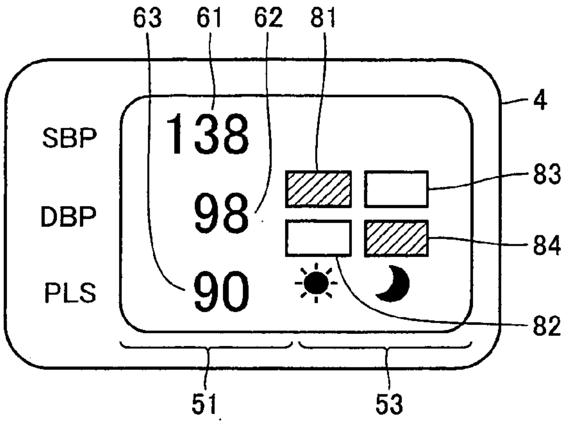


FIG.13A

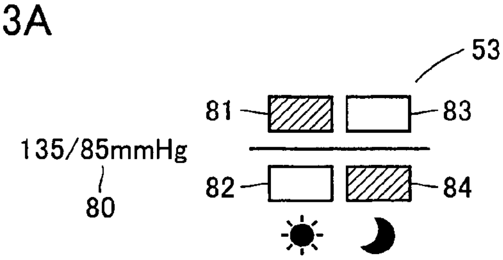


FIG.13B

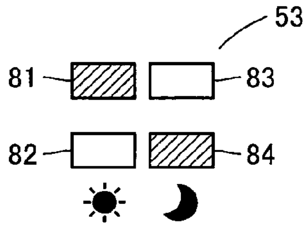


FIG.13C

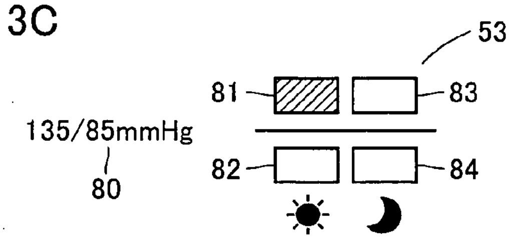


FIG.13D

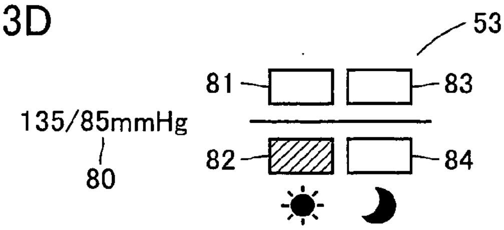

FIG.14

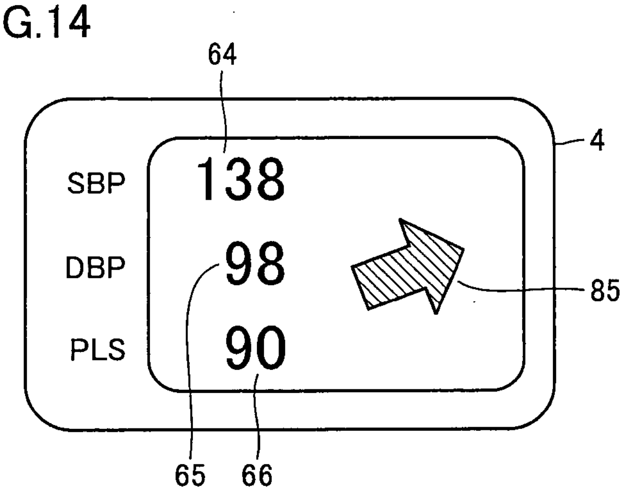


FIG.15

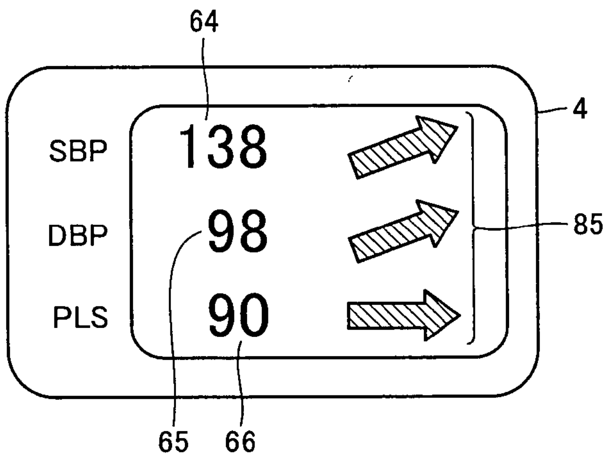


FIG.16

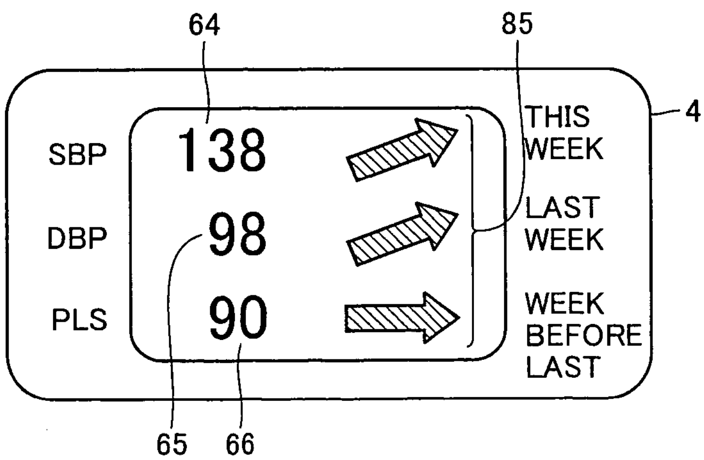

FIG.17

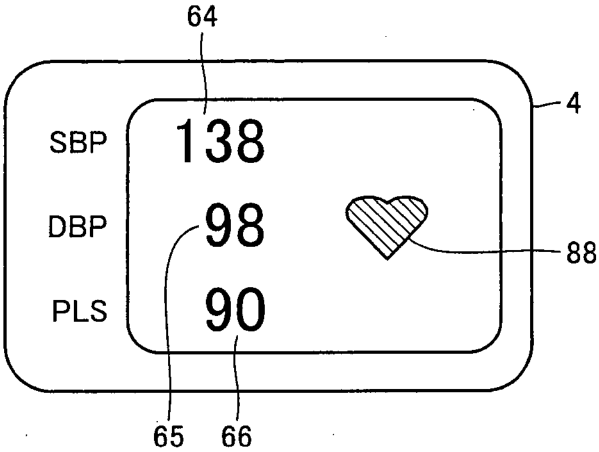


FIG.18

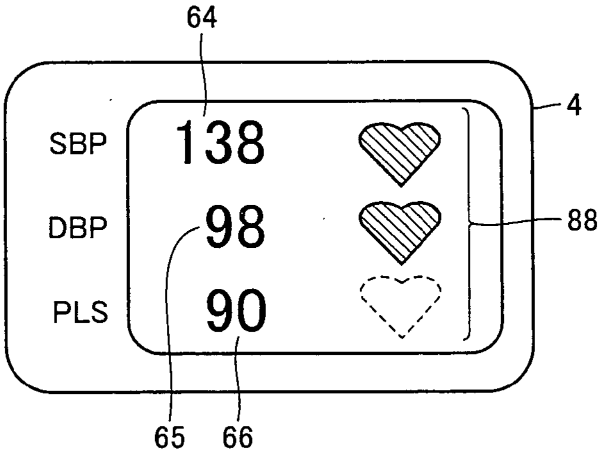


FIG.19

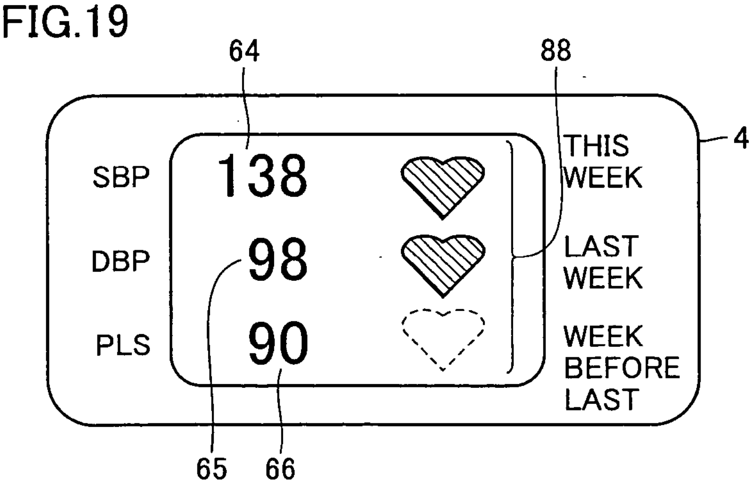

FIG.20

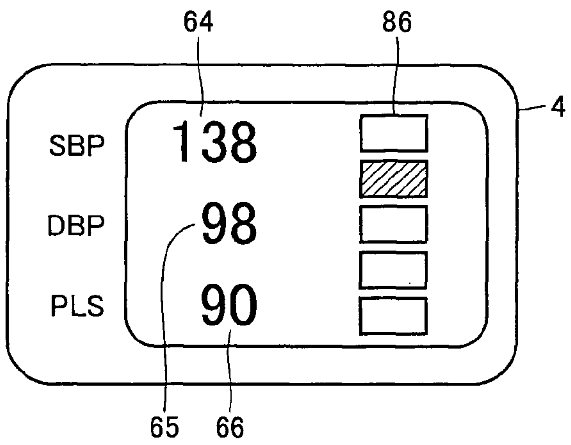


FIG.21

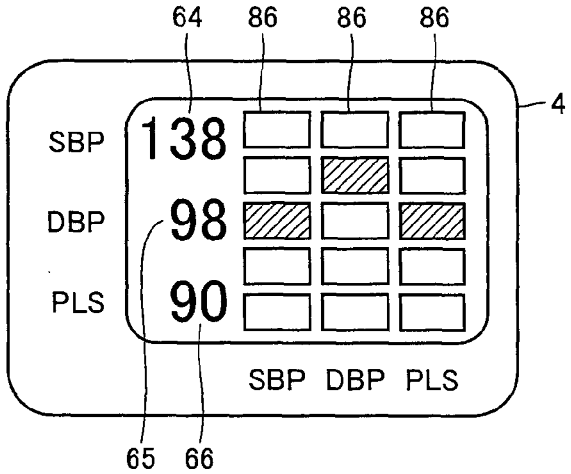


FIG.22

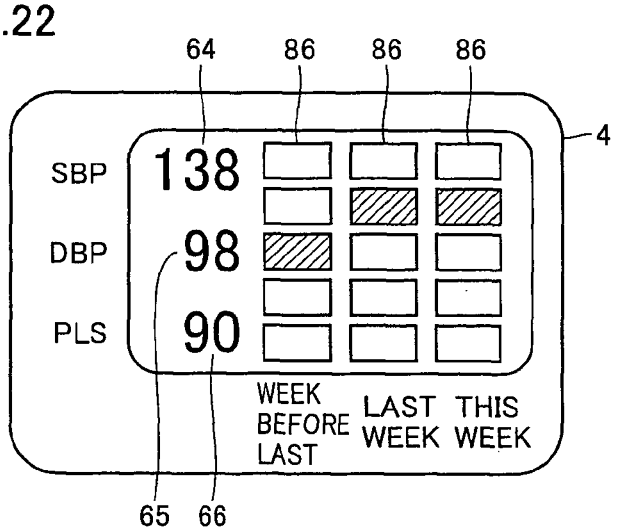

FIG.23

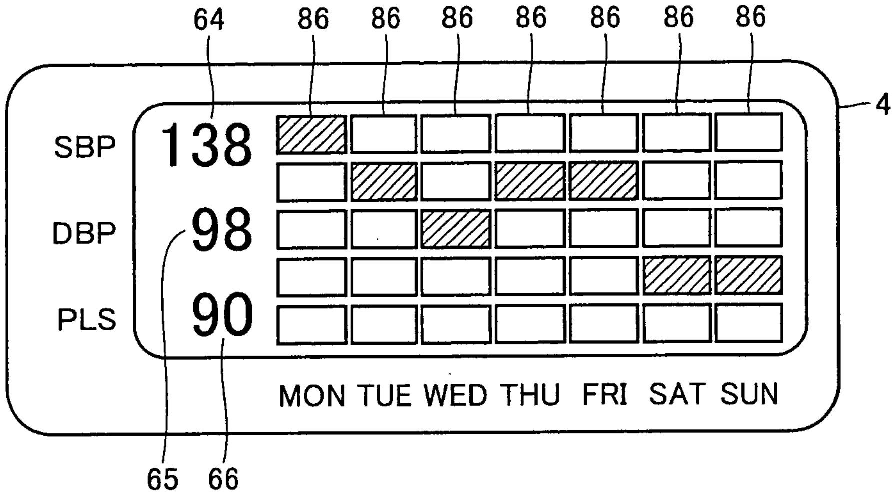


FIG.24A FIG.24B FIG.24C FIG.24D FIG.24E

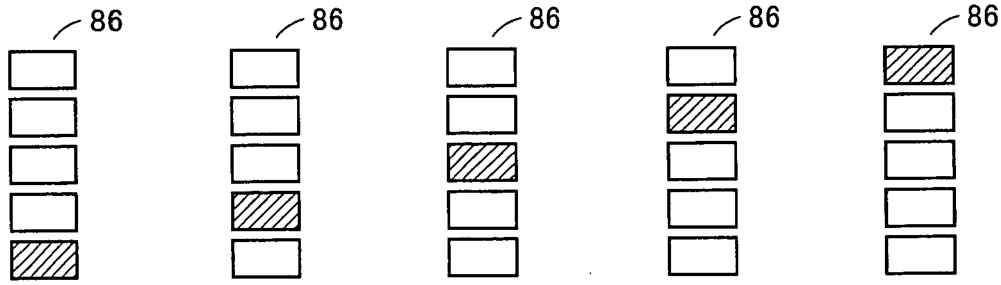


FIG.25

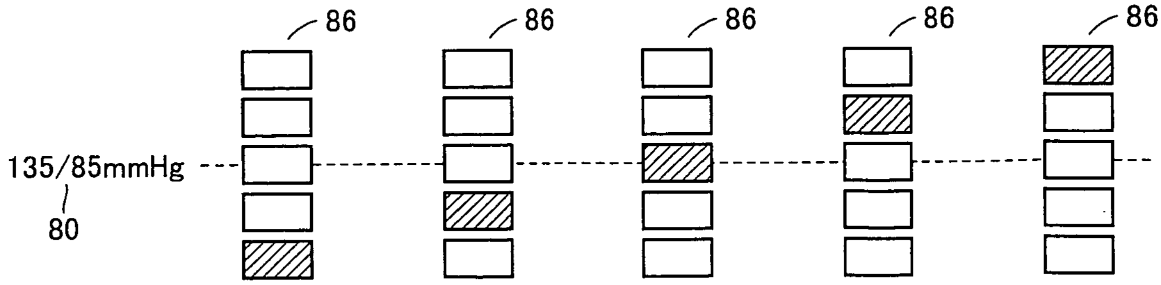

FIG.26

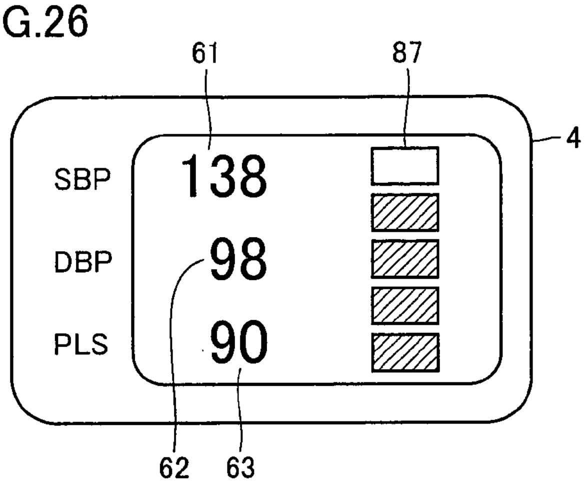


FIG.27

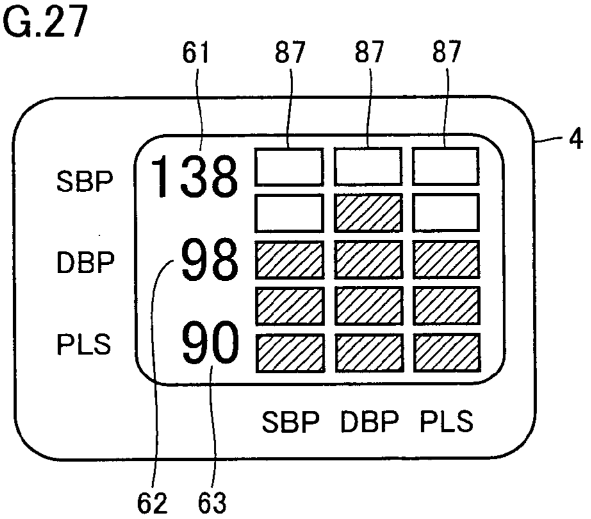


FIG.28

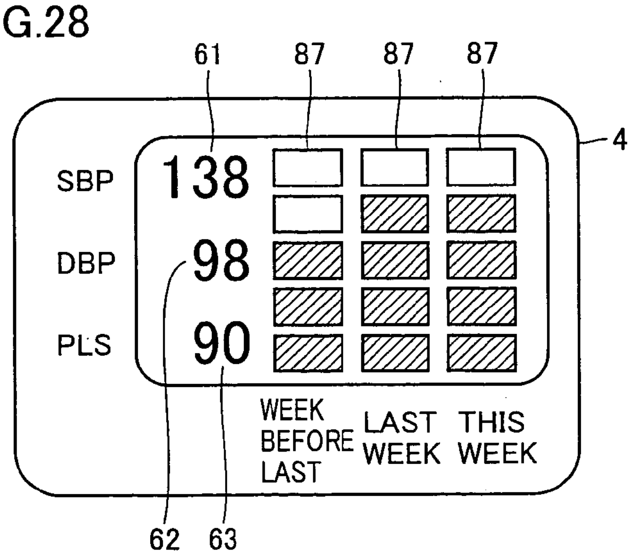

FIG.29

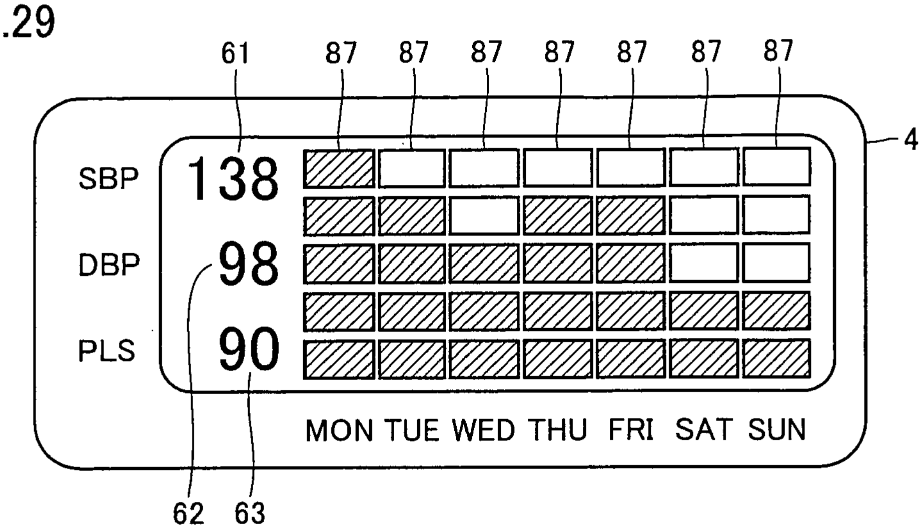


FIG.30A FIG.30B FIG.30C FIG.30D FIG.30E

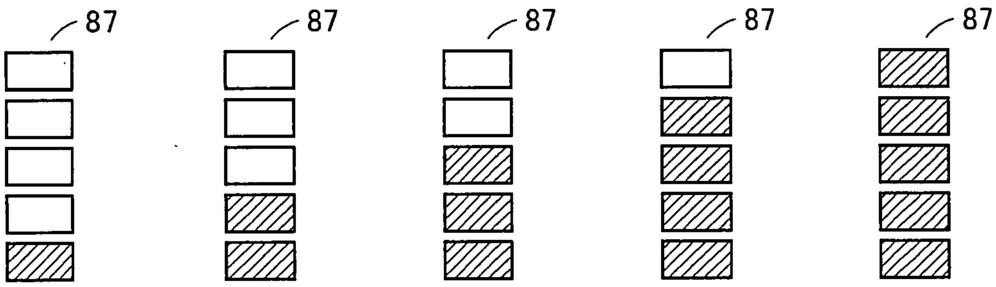


FIG.31

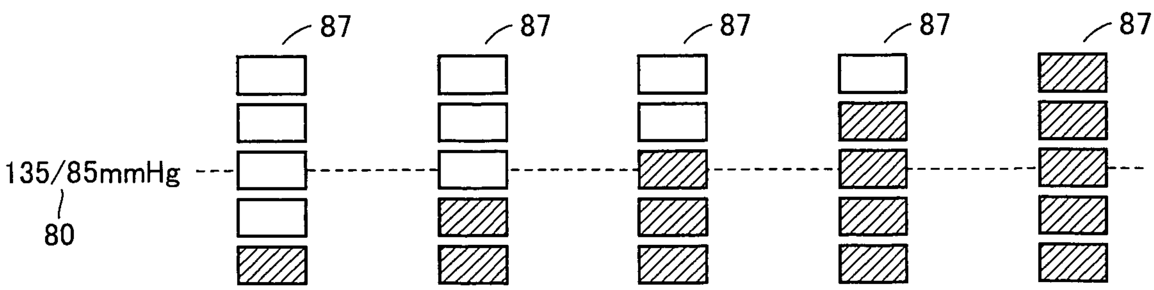

FIG.32

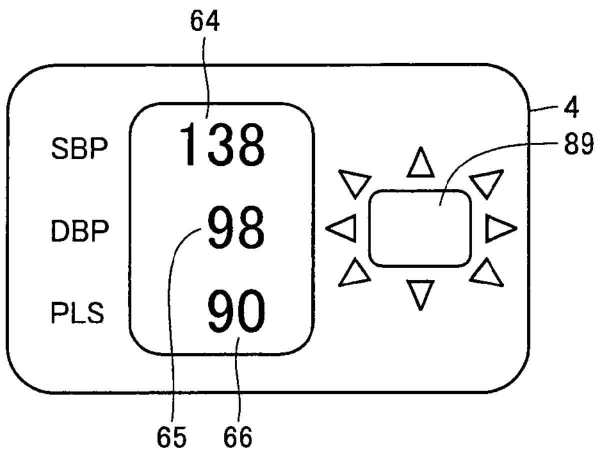


FIG.33

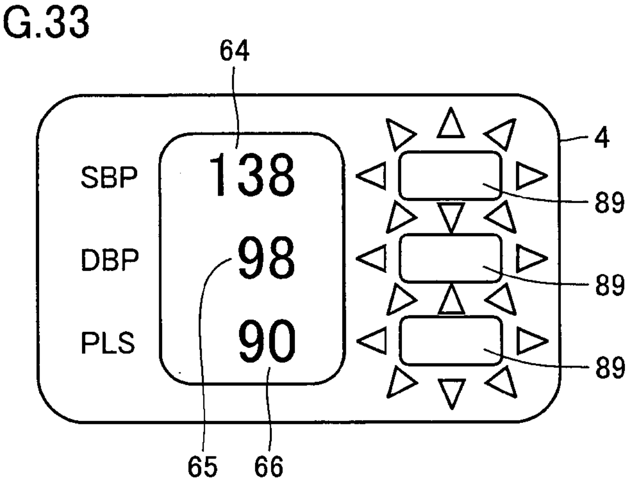


FIG.34

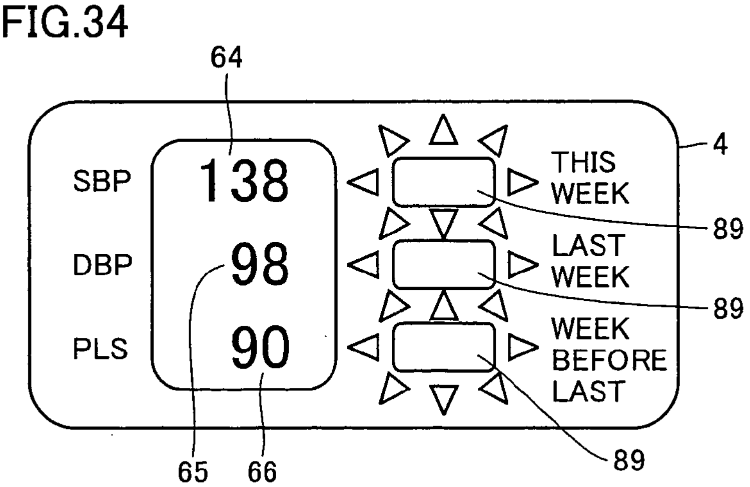

FIG.35A

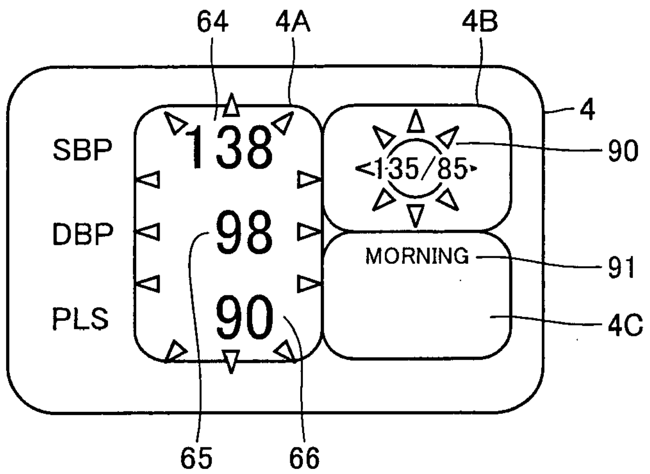


FIG.35B

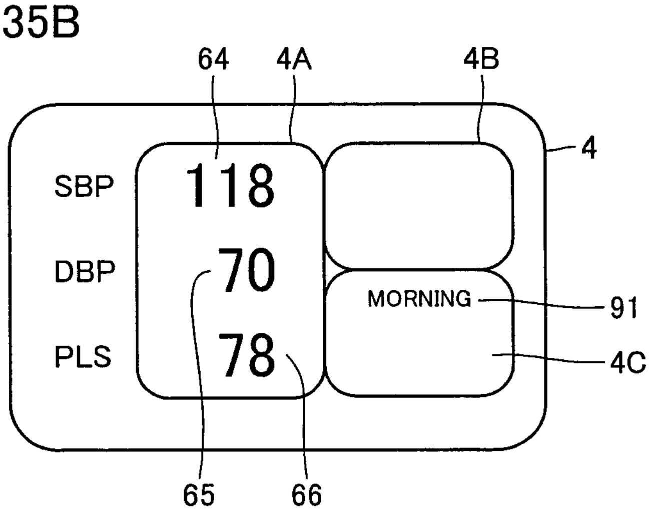

FIG.36A

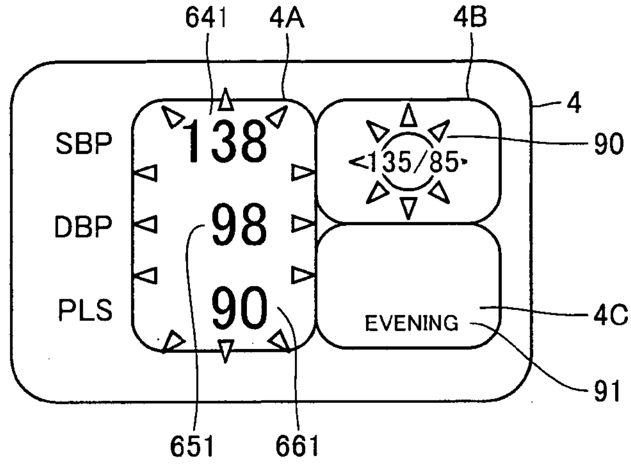


FIG.36B

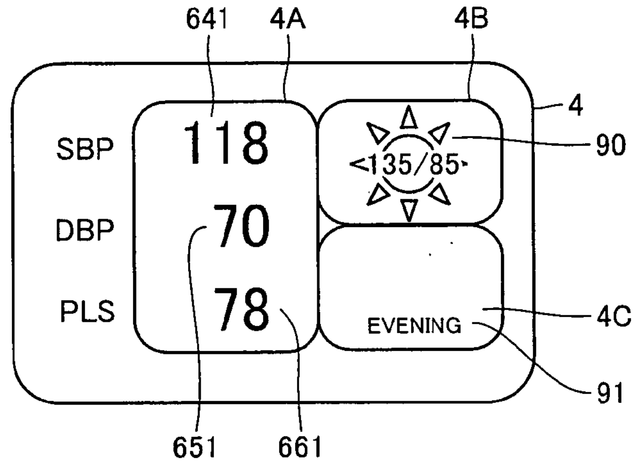


FIG.36C

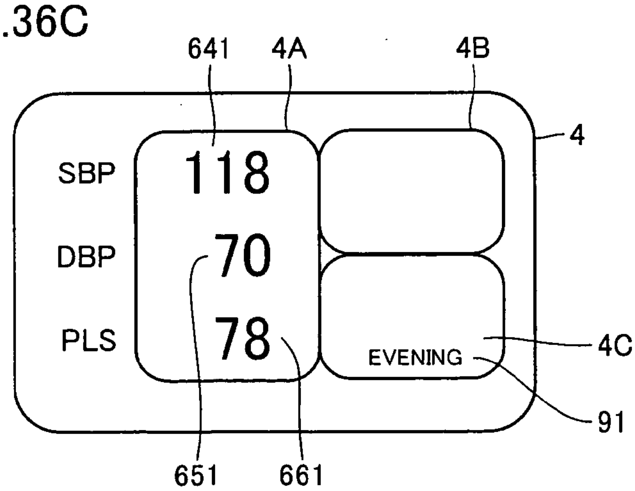

European Patent Office

# EUROPEAN SEARCH REPORT

Application Number
EP 06 01 6580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/176692 A1 (KARIO KAZUOMI [JP] ET AL) 9 September 2004 (2004-09-09)<br>* paragraph [0055] - paragraph [0068] *<br>* paragraph [0092] - paragraph [0108] *<br>* paragraph [0130] *<br>----- | 1-32 | INV.<br>A61B5/022 |
| X | US 4 995 399 A (HAYASHI RESSEI [JP] ET AL) 26 February 1991 (1991-02-26)<br>* column 5, line 15 - line 45 *<br>* column 6, line 58 - column 7, line 24 *<br>----- | 1-32 | |
| A | US 6 152 880 A (OKADA KOICHI [JP]) 28 November 2000 (2000-11-28)<br>* column 3, line 66 - column 4, line 64 *<br>* figures 6-10 *<br>----- | 1-32 | |
| A | US 2005/171442 A1 (SHIRASAKI OSAMU [JP] ET AL) 4 August 2005 (2005-08-04)<br>* paragraph [0047] *<br>* paragraph [0060] *<br>* paragraph [0103] - paragraph [0105] *<br>----- | 1-32 | |
| | | | TECHNICAL FIELDS SEARCHED (IPC) |
| | | | A61B |

The present search report has been drawn up for all claims

1

| Place of search | Date of completion of the search | Examiner |
|---|---|---|
| Berlin | 10 November 2006 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 06 01 6580

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2006

| Patent document cited in search report | Publication date | Patent family member(s) | Publication date |
|---|---|---|---|
| US 2004176692 A1 | 09-09-2004 | JP 2004261452 A | 24-09-2004 |
| US 4995399 A | 26-02-1991 | DE 68918202 D1 | 20-10-1994 |
| | | DE 68918202 T2 | 26-01-1995 |
| | | EP 0356016 A1 | 28-02-1990 |
| | | JP 2020507 U | 09-02-1990 |
| | | JP 5032082 Y2 | 18-08-1993 |
| US 6152880 A | 28-11-2000 | CN 1257690 A | 28-06-2000 |
| | | DE 29922408 U1 | 17-02-2000 |
| | | DE 69928753 T2 | 31-08-2006 |
| | | EP 1010392 A1 | 21-06-2000 |
| | | JP 2000175873 A | 27-06-2000 |
| US 2005171442 A1 | 04-08-2005 | CN 1650798 A | 10-08-2005 |
| | | EP 1561419 A2 | 10-08-2005 |
| | | JP 2005218492 A | 18-08-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004261452 A
- JP 2000175873 A
- JP 2004121632 A